Europäisches Patentamt

European Patent Office

Office européen des brevets

(11) Publication number: **0 249 676**
A2

(19)

(12) # EUROPEAN PATENT APPLICATION

(21) Application number: **87101137.5**

(22) Date of filing: **28.01.87**

(51) Int. Cl.⁴: **C 12 N 15/00,** C 12 P 21/02,
C 12 N 1/20, C 12 N 5/00,
A 01 H 1/00

(30) Priority: **28.01.86 DK 412/86**

(43) Date of publication of application: **23.12.87**
**Bulletin 87/52**

(84) Designated Contracting States: **AT BE CH DE ES FR GB
GR IT LI LU NL SE**

(71) Applicant: **AKTIESELSKABET DE DANSKE
SUKKERFABRIKKER, Langebrogade 5,
DK-1411 Copenhagen K (DK)**

(72) Inventor: **Marcker, Kjeld Adrian, 1, Toftevej, DK-8250 Ega
(DK)**
Inventor: **Jensen, Jens Stougaard, 10, J.M. Morksgade 1.
sal, DK-8000 Arhus C (DK)**

(74) Representative: **Vossius & Partner,
Siebertstrasse 4 P.O. Box 86 07 67,
D-8000 München 86 (DE)**

(54) **Method for the expression of genes in plants.**

(57) A method for the expression of genes in plants, parts of
plants, and plant cell cultures, in which a DNA fragment is
used comprising an inducible plant promoter of root nodule-
specific genes, DNA-fragments comprising an inducible
plant promoter, to be used when carrying out the method,
said DNA-fragments being identical with, derived from or
comprising a 5' flanking region of root nodule-specific
genes of any origin as well as plasmids and transformed
Agrobacterium rhizogenes-strain which can be used when
carrying out the method.

0249676

A method for the expression of genes in plants, parts of plants, and plant cell cultures, and DNA fragments, plasmids, and transformed microorganisms to be used when carrying out the method, as well as the use thereof for the expression of genes in plants, parts of plants, and plant cell cultures.

The invention relates to a novel method for the expression of genes in plants, parts of plants, and plant cell cultures, as well as DNA fragments and plasmids comprising said DNA fragments to be used when carrying out the method. The invention furthermore relates to transformed plants, parts of plants and plant cells.

The invention relates to this method for the expression of genes of any origin under control of an inducible, root nodule specific promoter.

The invention relates especially to this method for the expression of root nodule-specific genes in transformed plants including both leguminous plants and other plants.

The invention relates furthermore to DNA fragments comprising an inducible plant promoter to be used when carrying out the method, as well as plasmids comprising said DNA fragments.

In the specification i.a. the following terms are used:

Root nodule-specific genes: Plant genes active only in the root nodules of leguminous plants, or

2

genes with an increased expression in root nodules. Root nodule-specific plant genes are expressed at predetermined stages of development and are activated in a coordinated manner during the symbiosis whereby a nitrogen fixation takes place and the fixed nitrogen is utilized in the metabolism of the plant.

Inducible plant promoter: Generally is meant a promoter-active 5' flanking region from plant genes inducible from a low activity to a high activity. In relation to the present invention "inducible plant promoter" means a promoter derived from, contained in or being identical with a 5' flanking region including a leader sequence of root nodule-specific genes and being capable of promoting and regulating the expression of a gene as characterised in relation to the present invention.

Leader sequence: Generally is meant a DNA sequence being transcribed into a mRNA, but not further translated into protein. The leader sequence comprises thus the DNA fragment from the start of the transcription to the ATG codon constituting the start of the translation. In relation to the present invention "leader sequence" means a short DNA fragment contained in the above inducible plant promoter and typically comprising 40-70 bp and which may comprise sequences being targets for a posttranscriptional regulation.

Promoter region: A DNA fragment containing a promoter which comprises target sequences for RNA polymerase as well as possible activation regions comprising target sequences for transcriptional effector substances. In the present invention, target sequences for transcriptional effectors may also be situated 3' to the promoter, i.e. in the coding sequences, the intervening sequences or on the 3' flanking region of a root nodule-specific gene.

Furthermore a number of molecular-biological terms generally known to persons skilled in the art are used, including the terms stated below:

CAP (addition) site: The nucleotide of the 5' end of the transcript where 7-methylGTP is added; In the Figures often given also as an asterisk *-marked nucleotide on a given nucleotide sequence.

DNA sequence or DNA segment: A linear array of nucleotides interconnected through phosphodiester bonds between the 3' and 5' carbon atoms of adjacent pentoses.

Expression: The process undergone by a structural gene to produce a polypeptide. It is a combination of transcription and translation as well as possible posttranslational modifications.

Flanking regions: DNA sequences surrounding coding regions. 5' flanking regions contain a promoter. 3' flanking regions may contain a transcriptional terminator etc.

Gene: A DNA sequence composed of three or four parts, viz. (1) the coding sequence for the gene

4

product, (2) the sequences in the promoter region which control whether or not the gene will be expressed, (3) those sequences in the 3' end conditioning the transcriptional termination and optionally polyadenylation, as well as (4) intervening sequences, if any.

Intervening sequences: DNA sequences within a gene which are not coding for any peptide fragment. The intervening sequences are transcribed into pre-mRNA and are eliminated by modification of pre-mRNA into mRNA. They are also called introns.

Chimeric gene: A gene composed of parts from various genes. E.g. the chimeric Lbc$_3$-5'-3'-CAT is composed of a chloroamphenicolacetyltransferase-coding sequence deriving from E. coli and 5' and 3' flanking regulatory regions of the Lbc$_3$ gene of soybean.

Cloning: The process of obtaining a population of organisms or DNA sequences deriving from one such organism or sequence by asexual reproduction, or more particular a process of isolating a particular organism or part thereof, and the propagation of this subfraction as a homogeneous population.

Coding sequences: DNA sequences determining the amino acid sequence of a polypeptide.

Cross-inoculation group: A group of leguminous plant species capable of producing functionally active root nodules with Rhizobium bacteria isolated from root nodules of other species of the group.

5

<u>Leghemoglobin (Lb)</u>: An oxygen-binding protein exclusively synthesized in root nodules. The Lb proteins regulate the oxygen partial pressure in the root nodule tissue and transport oxygen to the bacteroides. In this manner the oxygen-sensitive nitrogenase enzyme is protected. The Lb genes are root nodule-specific genes.

<u>Messenger-RNA (mRNA)</u>: RNA molecule produced by transcription of a gene and possibly modification of mRNA. The mRNA molecule mediates the genetic message determining the amino acid sequence of a polypeptide by part of the mRNA molecule being translated into said peptide.

<u>Downstream</u>: A position in a DNA sequence. It is defined relative to the transcriptional direction 5'- 3' of the gene relative to which the position is stated. The 3' flanking region is thus positioned downstream of the gene.

<u>Nucleotide</u>: A monomeric unit of DNA or RNA consisting of a sugar moiety (pentose), a phosphate, and a nitrogeneous heterocyclic base. The base is linked to the sugar moiety via a glycosidic bond (1' carbon of the pentose), and this combination of base and sugar is a nucleoside. The base characterises the nucleotide. The four DNA bases are adenine (A), guanine (G), cytosine (C), and thymine (T). The four RNA bases are A, G, C, and uracil (U).

<u>Upstream</u>: A position in a DNA sequence. It is defined relative to the transcriptional direction 5'- 3' of the gene relative to which the position

6

is stated. The 5' flanking region is thus positioned upstream of this gene.

Plant transformation: Processes leading to incorporation of genes in the genome of plant cells in such a manner that these genes are reliably inherited through mitosis and meiosis or in such a manner that these genes are only maintained for short periods.

Plasmid: An extra-chromosomal double-stranded DNA sequence comprising an intact replicon such that the plasmid is replicated in a host cell. When the plasmid is placed within a unicellular organism, the characteristics of that organism are changed or transformed as a result of the DNA of the plasmid. For instance a plasmid carrying the gene for tetracycline resistance ($Tc^R$) transforms a cell previously sensitive to tetracycline into one which is resistant to it. A cell transformed by a plasmid is called a transformant.

Polypeptide: A linear array of amino acids interconnected by means of peptide bonds between the $\alpha$-amino and carboxy groups of adjacent amino acids.

Recombination: The creation of a new DNA molecule by combining DNA fragments of different origin.

Homologous recombination: A recombination between sequences showing a high degree of homology.

Replication: A process reproducing DNA molecules.

Replicon: A self-replicating genetic element possessing an origin for the initiation of DNA replication and genes specifying the functions necessary for a control and a replication thereof.

Restriction fragment: A DNA fragment resulting from double-stranded cleavage by an enzyme recognizing a specific target DNA sequence.

RNA polymerase: Enzyme effecting the transcription of DNA into RNA.

Root nodule: Specialized tissue resulting from infection of mainly roots of leguminous plants with Rhizobium bacteria. The tissue is produced by the host plant and comprises therefore plant cells whereas the Rhizobium bacteria upon infection are surrounded by a plant cell membrane and differentiate into bacteroides. Root nodules are produced on other species of plants upon infection of nitrogen-fixing bacteria not belonging to the Rhizobium genus. Root nodule-specific plant genes are also expressed in these nodules.

Southern-hybridization: Denatured DNA is transferred upon size separation in agarose gel to a nitrocellulose membrane. Transferred DNA is analysed for a predetermined DNA sequence or a predetermined gene by hybridization. This process allows a binding of single-stranded, radioactively marked DNA sequences (probes) to complementary single-stranded DNA sequences bound on the membrane. The position of DNA fragments on the membrane binding the probe can subsequently be detected on an X-ray film.

8

Symbiotic nitrogen fixation: The relationship whereby bacteroides of root nodules convert the nitrogen (dinitrogen) of the air into ammonium utilized by the plant while the plant provides the bacteroides with carbon compounds as a carbon source.

Symbiont: One part of a symbiotic relationship, and especially Rhizobium is called the microsymbiont.

Transformation: The process whereby a cell is incorporating a DNA molecule.

Translation: The process of producing a polypeptide from mRNA or:
the process whereby the genetic information present in a mRNA molecule directs the order of specific amino acids during the synthesis of a polypeptide.

Transcription: The method of synthesizing a complementary RNA sequence from a DNA sequence.

Vector: A plasmid, phage DNA or other DNA sequences capable of replication in a host cell and having one or a small number of endonuclease recognition sites at which such DNA sequences may be cleaved in a determinable manner without loss of an essential biological function.

Traditional plant breeding is based on repeated crossbreeding of plant lines individually carrying desired qualities. The identification of progeny lines carrying all the desired qualities is a particularly time-consuming process as the biochemical

9

and genetic basis of the qualities is usually unknown. New lines are therefore chosen according to their phenotype, usually after a screening of many lines in field experiments.

Through the ages a direct connection has existed between the state of nutrition, i.e. the health, of the population and the agricultural possibility of ensuring a sufficient supply of assimilable nitrogen in order to obtain satisfactory yields. Already in the seventeenth century it was discovered that plants of the family leguminosae including beyond peas also beans, lupins, soybean, bird's-foot trefoil, vetches, alfalfa, sainfoin, and trefoil had an ability of improving crops grown on the habitat of these plants. Today it is known that the latter is due to the fact that the members of the plants of the family leguminosae are able to produce nitrogen reserves themselves. On the roots they carry bacteria with which they live in symbiosis.

An infection of the roots of these leguminous plants with <u>Rhizobium</u> bacteria causes a formation of root nodules able to convert atmospheric nitrogen into bound nitrogen, which is a process called nitrogen fixation.

Atmospheric nitrogen is thereby converted into forms which can be utilized by the host plant as well as by the plants later on growing on the same habitat.

In the nineteenth century the above possibility was utilized for the supply of nitrogen in order to achieve a novel increase of the crop yield.

The later further increases in the yield have, however, especially been obtained by means of natural fertilizers and nitrogen-containing synthetic fertilizers. The resulting pollution of the environment makes it desirable to provide alternative possibilities of ensuring the supply of nitrogen necessary for the best possible yields obtainable.

It would thus be valuable to make an improvement possible of the existing nitrogen fixation systems in leguminous plants as well as to allow an incorporation of nitrogen fixation systems in other plants.

The recombinant DNA technique and the plant transformation systems developed render it now possible to provide plants with new qualities in a well-controlled manner. These characteristics can derive from not only the same plant species, but also from all other prokaryotic or eukaryotic organisms. The DNA techniques allow further a quick and specific identification of progeny lines carrying the desired qualities. In this manner a specific plant line can be provided with one or more desired qualities in a quick and well-defined manner.

Correspondingly, plant cells can be provided with well defined qualities and subsequently be maintained as plant cell lines by means of known tissue culture methods. Such plant cells can be utilized for the production of chemical and biological products of particular interest such as dyes, flavours, aroma components, plant hormones, pharmaceutical

11

products, primary and secondary metabolites as well as polypeptides (enzymes).

A range of factors and functions necessary for biological production of a predetermined gene product are known. Both the initiation and regulation of transcription as well as the initiation and regulation of posttransscriptional processes can be characterised.

At the gene level it is known that these functions are mainly carried out by 5' flanking regions. A wide range of 5' flanking regions from prokaryotic and eukaryotic genes has been sequenced, and in view inter alia thereof a comprehensive knowledge has been provided of the regulation of gene expression and of the sub-regions and sequences being of importance for the regulation of expression of the gene. Great differences exist in the regulatory mechanism of prokaryotic and eukaryotic organisms, but many common features apply to the two groups.

The regulation of the expression of gene may take place on the transscriptional level and is then preferably exerted by regulating the initiation frequency of transscription. The latter is well-known and described inter alia by Benjamin Lewin, Gene Expression, John Wiley & Sons, vol. I, 1974, vol. II, Second Edition 1980, vol. III, 1977. As an alternative the regulation may be exerted at the posttransscriptional level, e.g. by the regulation of the frequency of the translation initiation, at the rate of the translation, and of the termination of the translation.

The present invention is based on the surprising finding that 5' flanking regions of root nodule-specific genes, exemplified by the 5' flanking region of the soybean leghemoglobin $Lbc_3$ gene, can be used for inducible expression of a foreign gene in an alien leguminous plant. The induction and regulation of the promoter is preferably carried out in the form of a regulation and induction at the transscriptional level and differs thereby from the inducability stated in Patent Application No. 86114704.9, the latter inducability preferably being carried out at the translation level.

The transscription of both the $Lbc_3$ gene of the soybean and of a chimeric $Lbc_3$ gene transferred to bird's-foot trefoil starts at a low level immediately upon the appearance of the root nodules on the plant roots. Subsequently, a high increase of the transscription takes place immediately before the root nodules turn red. The transcription of a range of other root nodule-specific genes is initiated exactly at this time. The simultaneous induction of the transscription of the Lb genes and other root nodule-specific genes means that a common DNA sequence(s) must be present for the various genes controlling this pattern of expression. Thus the leghemoglobin-$c_3$ gene is a representative of one class of genes and the promoter and the leader sequence, target areas for activation as well as the control elements of the organ specificity of the $Lbc_3$ gene are representatives of the control elements of a complete gene class.

The promoter of the 5' flanking regions of the Lb genes functions in soybeans and is responsible for the transcription of the Lb genes in root nodules. It is furthermore known, that the efficiency of both the transcription initiation and the subsequent translation initation on the leader sequence of the Lb genes is high as the Lb proteins constitute approximately 20% of the total protein content in root nodules.

The sequence of 5' flanking regions of the four soybean leghemoglobin genes Lba, $Lbc_1$, $Lbc_2$, and $Lbc_3$ appears from the enclosed sequence scheme, scheme 1, wherein the sequences are stated in such a manner that the homology between the four 5' flanking regions appears clearly.

In the sequence scheme "-" indicates that no base is present in the position in question. The names of the genes and the base position counted upstream from the ATG start codon are indicated to the right of the sequence scheme. Furthermore the important sequences have been underlined.

As it appears from the sequence scheme a distinct degree of homology exists between the four 5' flanking regions, and in the position 23-24 bp upstream from the CAP addition site they all contain a TATATAAA sequence corresponding to the "TATA" box which in eukaryotic cells usually are located a corresponding number of bp upstream from the CAP addition site. Furthermore a CCAAG sequence is present 64-72 bp upstream from the CAP addition site, said sequence corresponding to the "CCAAT"

14

box usually located 70-90 bp upstream from the CAP addition site. From the CAP addition site to the translation start codon, ATG, leader sequences of 52-59 bp are present and show a distinct degree of homology of approx. 75-80%.

In accordance with the present invention it has furthermore been proved, exemplified by the $Lbc_3$ gene, that the 5' flanking regions of the soybean leghemoglobin genes are functionally active in other plant species. The latter has been proved by fusioning the E. coli chloroamphenicol acetyl transferase (CAT) gene with the 5' and 3' flanking regions of the soybean $Lbc_3$ gene in such a manner that the expression of the CAT gene is controlled by the Lb promoter. This fusion fragment was cloned into the integration vectors pAR1 and pAR22, whereby the plasmids pAR29 and pAR30 were produced. Through homologous recombination the latter plasmids were integrated into the Agrobacterium rhizogenes T DNA region. The transformation of Lotus corniculatus (bird's-foot trefoil) plants, i.e. transfer of the T DNA region, was obtained by wound infection on the hypokotyl. Roots developed from the transformed plant cells were cultivated in vitro and freed from A. rhizogenes bacteria by means of antibiotics. Completely regenerated plants were produced by these root cultures in a conventional manner through somatic embryogenesis or organogenesis.

Regenerated plants were subsequently inoculated with Rhizobium loti bacteria and root nodules for analysis were harvested. Transcription and translation of the chimeric $Lbc_3$ CAT gene could subse-

15

quently be detected in root nodules on transformed plants as the activity of the produced chloroamphenicol acetyl transferase enzyme.

The conclusion can subsequently be made that the promoter-containing 5' flanking regions of root nodule-specific genes exemplified by the soybean $Lbc_3$ promoter are functionally active in foreign plants. The latter is a surprising observation as root nodules are only developed as a consequence of a very specific interaction between the leguminous plant and its corresponding Rhizobium microsymbiont.

Soybeans produce nodules only upon infection by the species Rhizobium japonicum and Lotus corniculatus only upon infection by the species Rhizobium loti. Soybean and Lotus corniculatus belong therefore to two different cross-inoculation groups, each group producing root nodules by means of two different Rhizobium species. The expression of a chimeric soybean gene in Lotus corniculatus proves therefore an unexpected universal regulatory system applying to the expression of root nodule-specific genes. The regulatory DNA sequences involved can be placed on the 5' and 3' flanking regions of the genes, here exemplified by the 2.0 Kb 5' and 0.9 Kb 3' flanking regions of the $Lbc_3$ gene. This surprising observation allows the use of root nodule-specific promoters and regulatory sequences in any other plant species and any other plant cell line.

In other experiments the 5' flanking region of the nodule-specific N23 gene was fused to the CAT gene

16

and the Lbc₃ 3' flanking region in such a manner that the expression of the CAT gene is controlled by the N23 promoter. This fusion fragment was cloned into the integration vector pAR22 producing the plasmid N23-CAT which was subsequently recombined into A.rhizogenes and transferred to Lotus corniculatus and Trifolium repens (white clover) by the previously described method. The root nodule-specific expression of the transferred N23-CAT gene obtained in L. corniculatus infected with Rhizobium loti and in T.repens infected with Rhizobium trifolii further demonstrated that expression of root nodule-specific genes is independent of the plant species and Rhizobium species. A universal regulatory system therefore regulates the expression of root nodule-specific genes in the different symbiotic systems formed between legumes and the Rhizobium species of the various cross-inoculation groups.

It is known from European Patent Application EP 122,791.A1 that plant genes from one species, by Agrobacterium mediated transformation, can be transferred into a different plant species. It is also known from EP 122,791.A1 that a transferred gene encoding the seed storage protein "Phaseolin" can be expressed into tobacco and alfalfa. From the literature it is also known that this expression is seed specific (Sengupta-Gopalan et al. 1985, Proc. Natl. Acad. Sci. 82, 33203324).

The present invention therefore relates to a novel method for the expression of transferred genes in a root nodule-specific manner, using DNA regulatory sequences from the 5' promoter region, the coding region, or the 3' flanking region of root nodule-specific genes, here exemplified by the leghemoglobin $Lbc_3$ gene and the N23 gene. This method is distinct from both the method of <u>Agrobacterium</u> mediated transformation and expression of the <u>seed storage protein phaseolin</u> gene characterised in EP 122,791.A1. Expression of the transferred phaseolin gene in EP 122,791.A1 <u>only</u> demonstrates that the phaseolin gene family with its <u>particular</u> regulatory requirements can be expressed in tobacco and alfalfa. It does not demonstrate nor predict that <u>any</u> other genes with their particular regulatory requirements can be expressed in any other plants or plant tissue.

An object of the present invention is to provide a possibility of expressing desired genes in plants, parts of plants, and plant cell cultures.

A further object of the invention is to render it possible to express genes of any origin by the control of an inducible root nodule-specific promoter.

A particular object of the invention is to provide a possibility of expressing desired genes in leguminous plants.

A still further particular object of the invention is to provide a possibility of expressing root nodule-specific genes in non-leguminous plants.

Further objects of the invention are to improve the

18

existing nitrogen-fixing systems in leguminous plants as well as to incorporate nitrogen-fixing systems in other plants.

A further object of the invention is to provide a possibility of in certain cases allowing the use of specific sequences of the 3' flanking region, of the coding sequence, and of intervening sequences to influence the regulation of the root nodule-specific promoter.

Furthermore it is an object of the invention to provide plasmids comprising the above mentioned inducible plant promoter.

Further objects of the invention appear immediately from the following description.

The method according to the invention for the expression of genes in plants, parts of plants, and plant cell cultures is carried out by introducing into a cell thereof a recombinant DNA segment containing both the gene to be expressed and a 5' flanking region comprising a promoter sequence, and optionally a 3' flanking region, and culturing of the transformed cells in a growth medium, said method being characterised by using as the recombinant DNA segment a DNA fragment comprising an inducible plant promoter (as defined) from root nodule-specific genes. If desired the transformed cells are regenerated to plants.

The method according to the invention allows in a well defined manner an expression of foreign genes in plants, parts of plants, and plant cell cultures, in this connection especially genes providing the plants with desired properties such as for instance a resistance to plant diseases and increased content of valuable polypeptides.

A further use is the preparation of valuable products such as for instance dyes, flavourings, plant hormones, pharmaceutical products, primary and secondary metabolites, and polypeptides by means of the method according to the invention in plant cell cultures and plants.

By using the method according to the invention for the expression of root nodule-specific genes it is possible to express root nodule-specific genes necessary for the formation of an active nitrogen-fixing system both in leguminous plants and other plants. The correct developmental control, cf. Example 8, allows the establishment of a symbiotic nitrogen-fixing system in non-leguminous plants. In this manner it is surprisingly possible to improve the existing nitrogen-fixing systems in leguminous plants as well as to incorporate nitrogen-fixing systems in other plants.

The use of the method according to the invention for the expression of foreign genes in root nodules renders it possible to provide leguminous plants with improved properties such as resistance to herbicides and resistance to diseases and pest.

According to a particular embodiment of the method according to the invention a DNA fragment is used

20

which comprises an inducible plant promoter and which is identical with, derived from, or comprises 5' flanking regions of leghemoglobins genes. In this manner the expression of any gene is obtained.

Examples of such DNA fragments are DNA fragments of the four 5' flanking regions of the soybean leghemoglobin genes, viz.

Lba with the sequence:

```
GAGATACATT ATAATAATCT CTCTAGTGTC TATTTATTAT TTTATCTGGT
GATATATACC TTCTCGTATA CTGTTATTTT TTCAATCTTG TAGATTTACT
TCTTTTATTT TTATAAAAAA GACTTTATTT TTTTAAAAAA AATAAAGTGA
ATTTTGAAAA CATGCTCTTT GACAATTTTC TGTTTCCTTT TTCATCATTG
GGTTAAATCT CATAGTGCCT CTATTCAATA ATTTGGGCTC AATTTAATTA
GTAGAGTCTA CATAAAATTT ACCTTAATAG TAGAGAATAG AGAGTCTTGG
AAAGTTGGTT TTTCTCGAGG AAGAAAGGAA ATGTTAAAAA CTGTGATATT
TTTTTTTTGG ATTAATAGTT ATGTTTATAT GAAAACTGAA AATAAATAAA
CTAACCATAT TAAATTTAGA ACAACACTTC AATTATTTTT TTAATTTGAT
TAATTAAAAA ATTATTTGAT TAAATTTTTT AAAAGATCGT TGTTTCTTCT
TCATCATGCT GATTGACACC CTCCACAAGC CAAGAGAAAC ACATAAGCTT
TGGTTTTCTC ACTCTCCAAG CCCTCTATAT AAACAAATAT TGGAGTGAAG
TTGTTGCATA ACTTGCATCG AACAATTAAT AGAAATAACA GAAAATTAAA
AAAGAAATAT G,
```

Lbc$_1$ with the sequence:

```
TTCTCTTAAT ACAATGGAGT TTTTGTTGAA CATACATACA TTTAAAAAAA
AATCTCTAGT GTCTATTTAC CCGGTGAGAA GCCTTCTCGT GTTTTACACA
CTTTAATATT ATTATATCCT CAACCCCACA AAAAAGAATA CTGTTATATC
TTTCCAAACC TGTAGATTTA TTTATTTATT TATTTATTTT TACAAAGGAG
ACTTCAGAAA AGTAATTACA TAAAGATAGT GAACATCATT TTATTTATTA
TAATAAACTT TAAAATCAAA CTTTTTTATA TTTTTTGTTA CCCTTTTCAT
TATTGGGTGA AATCTCATAG TGAAGCCATT AAATAATTTG GGCTCAAGTT
TTATTAGTAA AGTCTGCATG AAATTTAACT TAACAATAGA GAGAGTTTTC
GAAAGGGAGC GAATGTTAAA AAGTGTGATA TTATATTTTA TTTCGATTAA
TAATTATGTT TACATGAAAA CATACAAAAA AATACTTTTA AATTCAGAAT
AATACTTAAA ATATTTATTT GCTTAATTGA TTAACTGAAA ATTATTTGAT
TAGGATTTTG AAAAGATCAT TGGCTCTTCG TCATGCCGAT TGACACCCTC
CACAAGCCAA GAGAAACTTA AGTTGTAAAC TTTCTCACTC CAAGCCTTCT
ATATAAACAT GTATTGGATG TGAAGTTATT GCATAACTTG CATTGAACAA
TAGAAAATAA CAAAAAAAAG TAAAAAAGTA GAAAGAAAT ATG,
```

21

Lbc$_2$ with the sequence:

```
TCGAGTTTTT ACTGAACATA CATTTATTAA AAAAAACTCT CTAGTGTCCA
TTTATTCGGC GAGAAGCCTT CTCGTGCTTT ACACACTTTA ATATTATTAT
ATCCCCACCC CCACCAAAAA AAAAAAAACT GTTATATCTT TCCAGTACAT
TTATTTCTTA TTTTTACAAA GGAAACTTCA CGAAAGTAAT TACAAAAAAG
ATAGTGAACA TCATTTTTTT AGTTAAGATG AATTTTAAAA TCACACTTTT
TTATATTTTT TTGTTACCCT TTTCATTATT GGGTGAAATC TCATAGTGAA
ACTATTAAAT AGTTTGGGCT CAAGTTTTAT TAGTAAAGTC TGCATGAAAT
TTAACTTAAT AATAGAGAGA GTTTTGGAAA GGTAACGAAT GTTAGAAAGT
GTGATATTAT TATAGTTTTA TTTAGATTAA TAATTATGTT TACATGAAAA
TTGACAATTT ATTTTTAAAA TTCAGAGTAA TACTTAAATT ACTTATTTAC
TTTAAGATTT TGAAAGATC ATTTGGCTCT TCATCATGCC GATTGACACC
CTCCACAAGC CAAGAGAAAC TTAAGTTGTA ATTTTTCTAA CTCCAAGCCT
TCTATATAAA CACGTATTGG ATGTGAAGTT GTTGCATAAC TTGCATTGAA
CAATAGAAAT AACAACAAAG AAAATAAGTG AAAAAAGAAA TATG,
```

and Lbc$_3$ with the sequence:

```
TATGAAGATT AAAAAATACA CTCATATATA TGCCATAAGA ACCAACAAAA
GTACTATTTA AGAAAGAAA AAAAAAACCT GCTACATAAT TTCCAATCTT
GTAGATTTAT TTCTTTTATT TTTATAAAGG AGAGTTAAAA AAATTACAAA
ATAAAAATAG TGAACATCGT CTAAGCATTT TTATATAAGA TGAATTTTAA
AAATATAATT TTTTTGTCTA AATCGTATGT ATCTTGTCTT AGAGCCATTT
TTGTTTAAAT TGGATAAGAT CACACTATAA AGTTCTTCCT CCGAGTTTGA
TATAAAAAAA ATTGTTTCCC TTTTGATTAT TGGATAAAAT CTCGTAGTGA
CATTATATTA AAAAAATTAG GGCTCAATTT TTATTAGTAT AGTTTGCATA
AATTTTAACT TAAAAATAGA GAAAATCTGG AAAAGGGACT GTTAAAAAGT
GTGATATTAG AAATTTGTCG GATATATTAA TATTTTATTT TATATGGAAA
CTAAAAAAAT ATATATTAAA ATTTTAAATT CAGAATAATA CTTAAATTAT
TTATTTACTG AAAATGAGTT GATTTAAGTT TTTGAAAAGA TGATTGTCTC
TTCACCATAC CAATTGATCA CCCTCCTCCA ACAAGCCAAG AGAGACATAA
GTTTTATTAG TTATTCTGAT CACTCTTCAA GCCTTCTATA TAAATAAGTA
TTGGATGTGA AGTTGTTGCA TAACTTGCAT TGAACAATTA ATAGAAATAA
CAGAAAAGTA GAAAGAAAT ATG.
```

A further embodiment of the method according to
the invention uses a DNA fragment identical with,
derived from or comprising 5' flanking regions of
the Lbc$_3$-5'-3'-CAT gene with the sequence:

```
TATGAAGATT AAAAAATACA CTCATATATA TGCCATAAGA ACCAACAAAA
GTACTATTTA AGAAAGAAA AAAAAAACCT GCTACATAAT TTCCAATCTT
GTAGATTTAT TTCTTTTATT TTTATAAAGG AGAGTTAAAA AAATTACAAA
ATAAAAATAG TGAACATCGT CTAAGCATTT TTATATAAGA TGAATTTTAA
AAATATAATT TTTTTGTCTA AATCGTATGT ATCTTGTCTT AGAGCCATTT
TTGTTTAAAT TGGATAAGAT CACACTATAA AGTTCTTCCT CCGAGTTTGA
```

22

```
TATAAAAAAA ATTGTTTCCC TTTTGATTAT TGGATAAAAT CTCGTAGTGA
CATTATATTA AAAAAATTAG GGCTCAATTT TTATTAGTAT AGTTTGCATA
AATTTTAACT TAAAAATAGA GAAAATCTGG AAAAGGGACT GTTAAAAAGT
GTGATATTAG AAATTTGTCG GATATATTAA TATTTTATTT TATATGGAAA
CTAAAAAAAT ATATATTAAA ATTTTAAATT CAGAATAATA CTTAAATTAT
TTATTTACTG AAAATGAGTT GATTTAAGTT TTTGAAAAGA TGATTGTCTC
TTCACCATAC CAATTGATCA CCCTCCTCCA ACAAGCCAAG AGAGACATAA
GTTTTATTAG TTATTCTGAT CACTCTTCAA GCCTTCTATA TAAATAAGTA
TTGGATGTGA AGTTGTTGCA TAACTTGCAT TGAACAATTA ATAGAAATAA
CAGAAAAGTA GAATTCTAAA ATG
```

A still further preferred embodiment of the method according to the invention uses a DNA fragment identical with, derived from or comprising 5' flanking regions of the N23 gene with the sequence

```
        10        20        30        40        50        60        70
GAATTCGAGCTCGCCCGGGGATCGATCCTCTAGAGTCGACCTGCAGCCCAAGCTTGGATCAATCAATTAA
EcoRI                              SalI

        80        90       100       110       120       130       140
TTCTATTGAGACACGATTTGAACAATTTTTACATTATGAGACTATTTTTGGTTTTTTATTTGATCCAAAA

       150       160       170       180       190       200       210
AAATTTAAAGCTTTAGATGATGATGAATTGAANNAATATTGTATTAATNNTGAAAAGTTNNNNNGGTTTA

       220       230       240       250       260       270       280
ATGAATGCTATGATATTGATGGTCTTGATNTATTNNCAGAATTGAAAGTATTAAGAGAAGTGTTAAGAAA

       290       300       310       320       330       340       350
AGAAGTTAGCACACCAATAGAAGTATTGAGTTATATTAAAACTTTAGATTCTTTTCAAATGTTTACATTG

       360       370       380       390       400       410       420
CATATAGAATTTTATTGACAATCCTTATAACAGTTGCTACTGTTGAAAGACGTTCTTCAAAAATTAAAATT

       430       440       450       460       470       480       490
ACTTAAATCATATCTAAAATCAACAATGTTACAAGATAGATTGAATGAGTTAGTTATTTTATCTATTGAA

       500       510       520       530       540       550       560
AGTAAAGTGTTAGAATTGTTTGATTATAAAACTCTGATAAATGATTTTGCAGTTAAAAAAAACTAGAAGAT

       570       580       590       600       610       620       630
TAATATAAAAATTGATATTTTATATAATATATTAAGTCTCTTTAAAATTCTTGTAAAAAAAGACATTTTT

       640       650       660       670       680       690       700
AAATAATAAAATAAAGCAACTCTTAATTTTAATGAAACATCCCTTTGTTAAACCGAATCTTCCATAATGT

       710       720       730       740       750       760       770
AAAAATTAATGCTTGATGGAAGTTTTTAATTTGTTCTACTCAATACTCAAAGGGTTGTAAATATTTTTTT

       780       790       800       810       820       830       840
TATCATTTATATGTTGTAAATATGAATGCACTAGTAATTAGTTTAATGATAAAATATATTCTACAGATAT
```

```
        850       860       870       880       890       900       910
ATTTCTGTCTCTTGGCAACTCGTGAGAATTGAATATATTATAAAGATGAAAGGTCGTTACAATTTTTTTT

        920       930       940       950       960       970       980
AGAATAAATATTTATATACAATTCCTAGATTTTGTTATAAAATTCACATATTGTATGAGTATAAATACAT

        990      1000      1010      1020      1030      1040      1050
GAGCACACACCAAACTAGTCTCAAATTAAGTAAGGTGCTAATTATTAGCGGCTAGCTAAGTAACCAAGTA
                                                       DdeI

ATTAATG
```

In a particularly preferred embodiment of the method according to the invention a 3' flanking region of root nodule-specific genes is furthermore used, in particular sequences of the 3' flanking region capable of influencing the activity or regulation of a promotor of the root nodule-specific genes or the transcription termination, or capable of influencing the yield of the desired gene product in another manner.

Examples of such 3' flanking regions are the four 3' flanking regions of the soybean leghemoglobin genes, viz.

Lba with the sequence:

```
                    1590                                      1620
            TAA TTA GTA TCT ATT GCA GTA AAG TGT AAT AAA TAA ATC TTG

                    1650                                      1680
    TTT CAC TAT AAA ACT TGT TAC TAT TAG ACA AGG GCC TGA TAC AAA ATG TTG GTT AAA ATA

                    1710                                      1740
ATG GAA TTA TAT AGT ATT GGA TAA AAA TCT TAA GGT TAA TAT TCT ATA TTT GCG TAG GTT

                    1770                                      1800
TAT GCT TGT GAA TCA TTA TCG GTA TTT TTT TTC CTT TCT GAT AAT TAA TCG GTA AAT TA

                    1830                                      1860
ACA AAT AAG TTC AAA ATG ATT TAT ATG TTT CAA AAT TAT TTT AAC AGC AGG TAA AAT GTT

ATT TGG TAC GAA AGC TAA TTC GTC GA
```

24

Lbc₁ with the sequence:

                                                                1320
                              TAA/TT AGG ATC TAC TGC ATT GCC GTA

                         1350                                    1380
AAG TGT AAT AAA TAA ATC TTG TTT CAA CTA AAA CTT GTT ATT AAA CAA GTT CCC TAT ATA

                         1410                                    1440
AAT GTT GTT TAA AAT AAG TAA ATT TCA TTG TAT TGG ATA AAC ACT TTT AAG TTA TAT ATT

                         1470                                    1500
TCC ATA TAT TTA CGT TTG TGA ATC ATA ATC GAT ACT TTA TAA AAA TAA ATT CCA AAT AAT

TTA TAC GTT TTA AAA ATT ATT TT


Lbc₂ with the sequence:

                                   TAG/GAT CTA CTA TTG CCG TCA AGT
                                                              1140

GTA ATA AAT AAA TTT TGT TTC ACT AAA ACT TGT TAT TAA ACA AGT CCC CGA TAT ATA AAT
                        1170                                    1200
GTT GGT TAA AAT AAG TAA ATT ATA CGG TAT TGA TAA ACA ATC TTA AGT TTT ATA TAT AGT
                        1230                                    1260

TCC ATA TAC TAA AGT TTG TGA ATC ATA ATC GA
                        1290


and Lbc₃ with the sequence:

                         TAG/GAT CTA CAA TTG CCT TAA AGT GTA ATA AAT AAA
                         990                                    1020

TAT TAT TTC ACT AAA ACT TGT TAT TAA ACC AAG TTC TCG ATA TAA ATG TTG GTT AAA CTA
                        1050                                    1080

AGT AAA TTA TAT GGT ATT GGA TAA ACA ATC TTA AGC TT
                        1110


This sequence is positioned on the 0.9 Kb 3' flanking region used according to the invention. A
particular embodiment of the invention is therefore

25

the use of sequences of this region exerting or mediating the regulation characterised by the invention of root nodule-specific promoter regions.

In a preferred embodiment of the method according to the invention a region is used of the coding sequence or intervening sequence of root nodule-specific genes, in particular sequences of the coding sequence or the intervening sequence capable of influencing the regulation of a promotor of the root nodule-specific genes or capable of influencing the yield of the desired gene product in another manner.

Examples of such coding sequences and intervening sequences are the four leghemoglobin genes of soybean, viz.

Lba with the sequence:

```
                                                              120
                                                              VAL
                                                              ATG/GTT


                                         150                                      180
ALA PHE THR GLU LYS GLN ASP ALA LEU VAL SER SER SER PHE GLU ALA PHE LYS ALA ASN
GCT TTC ACT GAG AAG CAA GAT GCT TTG GTG AGT AGC TCA TTC GAA GCA TTC AAG GCA AAC

                                         210                                      240
ILE PRO GLN TYR SER VAL VAL PHE TYR THR SER
ATT CCT CAA TAC AGC GTT GTG TTC TAC ACT TC/G TAA GTT TTC TCT CTA AGC ATG TGT CTT

                                         270                                      300
CCA TTC TAT GTT TTT CTT TTG GAA ATT TGT TGT GTT TGA AAA AAG ATA TAT TGT TAA TGT

                                         330                                      360
                                       ILE LEU GLU LYS ALA PRO ALA ALA LYS ASP
GAG TGG TTT TGG TTT GAT TAA AAA TGA ATAG/G ATA CTG GAG AAA GCA CCT GCA GCA AAG GAC

                                         390                                      420
LEU PHE SER PHE LEU ALA ASN GLY VAL ASP PRO THR ASN PRO LYS LEU THR GLY HIS ALA
TTG TTC TCA TTT CTA GCA AAT GGA GTA GAC CCC ACT AAT CCT AAC CTC ACG GGC CAT GCT

                                         450                                      480
GLU LYS LEU PHE ALA LEU
GAA AAG CTT TTT GCA TTG/GTAA GTA TCA CCC AAC TAA AAT TAT AAC TAT TTT ATG TGA

                                         510                                      540
TTA ATT TTA AGA TTA AGC ATC ATG TAT TTT AAC ACT CTT AAA ACA TCA ATG AAC ATT AAT

                                         570                                      600
TGT TTG AAT TGT ATT TTA TAT TTT TGC CAT ATC TTG AAC TAG GAA TAG TAT ATA AAT TTC

                                         630                                      660
TAT TAG TAT TTG TTG ATA ATT ATT TTT CTT TCA TAA CTA TCT TGT CAC ATA TTA TAT ATT
```

```
                                              690                                                    720
                      VAL ARG ASP SER ALA GLY GLN LEU LYS ALA SER GLY THR VAL VAL ALA
        TTT TGA ATT GTAG/GTG CGT GAC TCA GCT GGT CAA CTT AAA GCA AGT GGA ACA GTG GTG GCT

                                              750                                                    780
        ASP ALA ALA LEU GLY SER VAL HIS ALA GLN LYS ALA VAL THR ASP PRO GLN PHE VAL
        GAT GCC GCA CTT GGT TCT GTT CAT GCC CAA AAA GCA GTC ACT GAT CCT CAG TTC GTG/GT

                                              810                                                    840
        ATG ATA AAT AAT GAA ATG TTA TAA TAA ATT ATG CAT ACT TCA ATT TTT CAT GGA GCA GTA

                                              870                                                    900
        TAA TGA TCA ACA CAC ACT TCT TTT GTT TCA TGC ATT TGA TAA CTA CAA TCT TAA AAT GTT

                                              930                                                    960
        GCA ATC TTA AAA ATA GTA TTA AAA ATA TAA CAT TTA ATT AGC TCA TCA ATA TTT TTC TGT

                                              990                                                   1020
        TGC AAT TTT TTA TGA AAA AAT TAT AAT TAT GAA TTC TTT GAG CAA TGT TTA ATT AAA AAA

                                             1050                                                   1080
        TTG ATT TAA TAA TGA AAT AAC TAA GCT ACC TCT GTC TCG TTT TTC ATT TAA ACT ATG ACA

                                             1110                                                   1140
        TAA ACA ATG AAT AAA GTA AAC TAA ACC ATG ACA TGT TTA TTT TTG AAT GAG GTT ATT AAT

                                             1170                                                   1200
        AAT TTT TTT TCA CTA TCT ATT GCA ATG TTC ATT GAT TAT CAA TTA TCT TGG TTG CAT TGA

                                             1230                                                   1260
        TTC TCT CGA TTT TTT TCT TGA GGT TAA GCT TCA GTT CAA TAT ATA TTC ATT TTT TGA TAA

                                             1290                                                   1320
        AAA AAA ATA GTA CAA TAT ATT TTC ATT TAG CTG ATC ATA TTT ATT TAA GTT CAA CTT AAA

                                             1350                                                   1380
        ATT TTA TAG ATG TTA ATT GAT ATA ATT TGT TGA GAT GAT GAG AAG ACC AAT ACC ATT ACG

                                             1410                                                   1440
        TAC TCT TTT GAA AGT GTT ATA TGG ATT TTA ATT ATA AGG AAA AAT GTA AGA GCT AAA CCA

                                             1470                                                   1500
                      VAL VAL LYS GLU ALA LEU LEU LYS THR ILE LYS ALA ALA VAL
        TTG CTG ATG ATT TTG AAG/GTG GTT AAA GAA GCA CTG CTG AAA ACA ATA AAG GCA GCA GTT

                                             1530                                                   1560
        GLY ASP LYS TRP SER ASP GLU LEU SER ARG ALA TRP GLU VAL ALA TYR ASP GLU LEU ALA
        GGG GAC AAA TGG AGT GAC GAG TTG AGC CGT GCT TGG GAA GTA GCC TAC GAT GAA TTG GCA

        ALA ALA ILE LYS LYS ALA
        GCA GCT ATT AAG AAG GCA TAA
```

The amino acid sequence of the Lba protein is indicated above the coding sequence,

Lbc$_1$ with the sequence:

27

```
                                                                        180
                                                                        GLY
                                                                      ATG/GCT

                               210                                      240
    ALA PHE THR GLU LYS GLN GLU ALA LEU VAL SER SER SER PHE GLU ALA PHE LYS ALA ASN
    GCT TTC ACT GAG AAG CAA GAG GCT TTG GTG AGT AGC TCA TTC GAA GCA TTC AAG GCA AAC

                            270                                         300
    ILE PRO GLN TYR SER VAL VAL PHE TYR ASN SER
    ATT CCT CAA TAC AGC GTT GTG TTC TAC AAT TC/GTAA GTT TTC TCT ATA AGC ATG TGT CTT

                               330                                      360
    TCA TTC TAT GTT TTT CTT CTG GAA ATT TTT TGT GTT TGA AAA AAG ATA TAT ATA TAT ATA

                            390                                         420
    TAT ATA TAT ATA TAT ATA TAT ATA TAT ATA TAT ATA TAT TTT GTT AAT GTG AGT GGT TTT

                               450                                      480
                         ILE LEU GLU LYS ALA PRO ALA ALA LYS ASP LEU PHE SER
    GGT TTG ATT AAA AAT AAA TAG/GATT CTG GAG AAA GCA CCT GCA GCA AAG GAC TTG TTC TCA

                            510                                         540
    PHE LEU ALA ASN GLY VAL ASP PRO THR ASN PRO LYS LEU THR GLY HIS ALA GLU LYS LEU
    TTT CTA GCA AAT GGA GTA GAC CCC ACT AAT CCT AAG CTC ACG GGC CAT GCT GAA AAG CTT

                            570                                         600
    PHE ALA LEU
    TTT GCA TTG/GT AAG TAT CAG CCA ACT AAA ATT ATA ACT ATT TTA TGT GAT TAA TTT TAA

                               630                                      660
    GAT TAA ACA TCA TGT ATT TTA ACA CTC TTA AAA TAT CAA TGA ACA TTA ATT TTT TGA ATT

                               690                                      720
    GTA TTT TAT ATT TTT ACC ATA TCT TGA ACT AGG AAT AAT ATA TAA ATT TCT ATT AGT ATT

                               750                                      780
    TGT TGG TAA TTA CAT ATA TAT ATA TAT ATA TAA TCC TTG TGA TAA TTA TTT TTC GAA TTT

                               810                                      840
            VAL ARG ASP SER ALA GLY GLN LEU LYS THR ASN GLY THR VAL VAL ALA ASP ALA ALA
    GTAG/GTG CGT GAC TCA GCT GGT CAA CTT AAA ACA AAT GGA ACA GTG GTG GCT GAT GCT GCA

                            870                                         900
    LEU VAL SER ILE HIS ALA GLN LYS ALA VAL THR ASP PRO GLN PHE VAL
    CTT GTT TCT ATC CAT GCC CAA AAA GCA GTC ACT GAT CCT CAG TTC GTG/GT ATG ATA AAT

                            930                                         960
    AAT ACT AGT AAA ATG TTA CAA TAA ATG CAA ACT TAA GTT TTA CGT ACA TAG TGA TCA TGA

                            990                                        1020
    CTT CAT GCA TGG CTA TTA TTT TTT CAT ATT TAT TGA AGT CAA CTT AAA ATT TTG TAA ATA

                           1050                                        1080
    CAG ATC GAT GCT AGT AAT TTG TTG AGA TCA TGA GAA AAC GTA CCA CTA CTC CAA TAG CAT

                           1110                                        1140
    TAC TCA TTT TGA AAA TTG TAT AAC TGT GAT CTA ATT ATA AGG AAA AAG TGT ATA TAA GAG

                           1170                                        1200
                                 VAL VAL LYS GLU ALA LEU LEU LYS THR
    CTA ATC CAT TAT TAA TGT TTT TTA TAT TTT GTAG/GTG GTT AAA GAA GCA CTG CTG AAA ACA

                           1230                                        1260
    ILE LYS GLU ALA VAL GLY GLY ASN TRP SER ASP GLU LEU SER SER ALA TRP GLU VAL ALA
    ATA AAG GAA GCT GTT GGC GGC AAT TGG AGT GAC GAA TTG AGC AGT GCT TGG GAA GTA GCC

                           1290
    TYR ASP GLU LEU ALA ALA ALA ILE LYS LYS ALA
    TAT GAT GAA TTG GCA GCA GCA ATT AAA AAG GCA TAA
```

The amino acid sequence of the Lbc$_1$ protein is
indicated above the coding sequence,

28

Lbc$_2$ with the sequence:

```
                                                                      GLY
                                                                     G/GGT
                                                                      180
    ALA PHE THR GLU LYS GLN GLU ALA LEU VAL SER SER SER PHE GLU ALA PHE LYS ALA ASN
    GCT TTC ACT GAG AAG CAA GAG GCT TTG GTG AGT AGC TCA TTC GAA GCA TTC AAG GCA AAC
                              210                                           240

    ILE PRO GLN TYR SER VAL VAL PHE TYR THR SER
    ATT CCT CAA TAC AGC GTT GTG TTC TAC ACT TC/GTA AGT TTT CTC TTA AAG CAT GTA TCT
                              270                                           300

    TTC ATT CTC TGT TTT TCC TTT CGA CAT TTT TTG TGT TTG AAA AGA GAT AGT GTC AAT GTG
                              330                                           360

                                        ILE LEU GLU LYS ALA PRO ALA ALA LYS
    AGT GGG TAT TTT TTT TTA TTA AAA ATT AAC AG/G ATA CTG GAG AAA GCA CCC GCA GCA AAG
                              390                                           420

    ASP LEU PHE SER PHE LEU SER ASN GLY VAL ASP PRO SER ASN PRO LYS LEU THR GLY HIS
    GAC TTG TTC TCG TTT CTA TCT AAT GGA GTA GAT CCT AGT AAT CCT AAG CTC ACG GGC CAT
                              450                                           480

    ALA GLU LYS LEU PHE GLY LEU
    GCT GAA AAG CTT TTT GGA TTG/GTA AGT ATC ATC CAA CTA AAA TTA TAG CTA TTT TAT GTG
                              510                                           540

    ATT AAT TTT AAG ATT AAA CAT GTA TTT AAC ACT CTT AAA CAT GTA TTT AAC ACT CTT AAG
                              570                                           600

    ATT AAA CAT GTA TTT AAC TAA AAC ATG TAT TTG CTG ATT ATT TTT TTT TTA TAA TTA TCT
                              630                                           660

                                        VAL ARG ASP SER ALA GLY GLN LEU LYS ALA
    TGT CAC ATA TTA TAT ATT TTT TGA ATT GTA G/GTG CGT GAC TCA GCT GGT CAA CTT AAA GCA
                              690                                           720

    ASN GLY THR VAL VAL ALA ASP ALA ALA LEU GLY SER ILE HIS ALA GLN LYS ALA ILE THR
    AAT GGA ACA GTA GTG GCT GAT GCC GCA CTT GGT TCT ATC CAT GCC CAA AAA GCA ATC ACT
                              750                                           780

    ASP PRO GLN PHE VAL
    GAT CCT CAG TTC GTG/GT ATG ATA AAT AAT AAA ATG TTA CAA TAA ATG CAC ATA TAC TTA
                              810                                           840

    AAT TTT ACA TGG TGC AGT GTT ATG ATC ATC ATT TTT GTT TAG TAA TGA ATT TAC TTA AAA
                              870                                           900

    TCT TAA ATT ATG TAC TTT TTG AAA GTT TTA TAT GGA ATT TTA ATT ATA GGG AAA AAT GTA
                              930                                           960

                                        VAL VAL LYS GLU ALA LEU LEU LYS THR
    AGA GCT AAT CCA TTA GTG ATG TTT TGT CTG TAG/GTG GTT AAA GAA GCA CTG CTG AAA ACA
                              990                                          1020

    LE LYS GLU ALA VAL GLY ASP LYS TRP SER ASP GLU LEU SER SER ALA TRP GLU VAL ALA
    ATA AAG GAG GCA GTT GGG GAC AAA TGG AGT GAT GAA TTG AGC AGT GCT TGG GAA GTA GCC
                             1050                                          1080

    TYR ASP GLU LEU ALA ALA ALA ILE LYS LYS ALA PHE
    TAT GAT GAA TTG GCA GCA GCT ATT AAG AAG GCA TTT TAC
                             1110
```

The amino acid sequence of the Lbc$_2$ protein is indicated above the coding sequence,

and Lbc$_3$ with the sequence:

```
                                                   GLY ALA PHE THR ASP
                                                 G/GGT GCT TTC ACT GAT
                                                                   120

    LYS GLN GLU ALA LEU VAL SER SER SER PHE GLU ALA PHE LYS THR ASN ILE PRO GLN TYR
    AAG CAA GAG GCT TTG GTG AGT AGC TCA TTT GAA GCA TTC AAG ACA AAC ATT CCT CAA TAC
                                    150                                     180

    SER VAL VAL PHE TYR THR SER
    AGT GTT GTG TTC TAC ACC TC/GTA AGT ATT CTA TCT AAA TTA TGT GTC TTA TTG TAT GTT
                                    210                                     240

    TAA CTT TCG TGG TTT GTT GTG TTT GAA AAA AAG ATA TAT ATT GTT AAT GTG AGT GGT TTT
                                    270                                     300

                                   ILE LEU GLU LYS ALA PRO VAL ALA LYS ASP LEU PHE SER
    GGT TTG ACT AAA AAT GAA TAG/G ATA CTG GAG AAA GCA CCT GTA GCA AAG GAC TTG TTC TCA
                                    330                                     360

    PHE LEU ALA ASN GLY VAL ASP PRO THR ASN PRO LYS LEU THR GLY HIS ALA GLU LYS LEU
    TTT CTA GCT AAT GGA GTA GAC CCC ACT AAT CCT AAG CTC ACG GGC CAT GCT GAA AAA CTT
                                    390                                     420

    PHE GLY LEU
    TTT GGA TTG/GT AAG TAT CCA GCC TAC TAA AAT TAA AAT CCT ATT AGT ATT TTT TAT TAT
                                    450                                     480

                                                             VAL ARG ASP SER
    TTT TCT TCC ATG ATT GTC TTG TCA CAT ATT ATA TAT TTT TTG AAT TAT AG/GTA CGT GAT TCA
                                    510                                     540

    ALA GLY GLN LEU LYS ALA SER GLY THR VAL VAL ILE ASP ALA ALA LEU GLY SER ILE HIS
    GCT GGT CAA CTT AAA GCA AGT GGA ACA GTG GTG ATT GAT GCC GCA CTT GGT TCT ATC CAT
                                    570                                     600

    ALA GLN LYS ALA ILE THR ASP PRO GLN PHE VAL
    GCC CAA AAA GCA ATC ACT GAT CCT CAA TTT GTG/G TAT GAT AAA TAA TGA AAA GCT ACA
                                    630                                     660

    ATA AAT GCA CAA ATA CTT AAT TTT ACA TAG TGC AGT GCT ATA TGA TCA TCA CTT TTG CTT
                                    690                                     720

    AGT AAT GAA TTT ACT TTT TTT TTT TAC AGA AGT AAT GGA TTT ACT TAA AAT CTT AAA TTA
                                    750                                     780

    TGT ACT TCT TTA AAG AGT TTT GTA TGG AAT TTT AAT TAT AGG AAA AAT GTA AGA GCT AAA
                                    810                                     840

                          VAL VAL LYS GLU ALA LEU LEU LYS THR ILE LYS GLU ALA
    CCA TTG CTG ATG ATT TCG AAG/GTG GTT AAA GAA GCA CTG CTG AAA ACA ATA AAG GAG GCA
                                    870                                     900

    VAL GLY ASP LYS TRP SER ASP GLU LEU SER SER ALA TRP GLU VAL ALA TYR ASP GLU LEU
    GTT GGG GAC AAA TGG AGT GAC GAG TTG AGC AGT GCT TGG GAA GTA GCC TAT GAT GAA TTG
                                    930                                     960

    ALA ALA ALA ILE LYS LYS ALA PHE
    GCA GCA GCT ATT AAG AAG GCA TTT TAG
```

The amino acid sequence of the Lbc$_3$ protein is indicated above the coding sequence.

The present invention furthermore deals with a novel DNA fragment comprising an inducible plant promoter to be used when carrying out the method according to the invention, said DNA fragment being characterised by being identical with, derived from or comprising a 5' flanking region of root nodule-specific genes. Examples of such DNA fragments are DNA fragments being identical with, derived from or comprising a 5' flanking region of plant leghemoglobin genes. Preferred examples are according to the invention DNA fragments being identical with, derived from or comprising a 5' flanking region of the four soybean leghemoglobin genes, viz.:

Lba with the sequence:

```
GAGATACATT ATAATAATCT CTCTAGTGTC TATTTATTAT TTTATCTGGT
GATATATACC TTCTCGTATA CTGTTATTTT TTCAATCTTG TAGATTTACT
TCTTTTATTT TTATAAAAAA GACTTTATTT TTTTAAAAAA AATAAAGTGA
ATTTTGAAAA CATGCTCTTT GACAATTTTC TGTTTCCTTT TTCATCATTG
GGTTAAATCT CATAGTGCCT CTATTCAATA ATTTGGGCTC AATTTAATTA
GTAGAGTCTA CATAAAATTT ACCTTAATAG TAGAGAATAG AGAGTCTTGG
AAAGTTGGTT TTTCTCGAGG AAGAAAGGAA ATGTTAAAAA CTGTGATATT
TTTTTTTTGG ATTAATAGTT ATGTTTATAT GAAAACTGAA AATAAATAAA
CTAACCATAT TAAATTTAGA ACAACACTTC AATTATTTTT TTAATTTGAT
TAATTAAAAA ATTATTTGAT TAAATTTTTT AAAAGATCGT TGTTTCTTCT
TCATCATGCT GATTGACACC CTCCACAAGC CAAGAGAAAC ACATAAGCTT
TGGTTTTCTC ACTCTCCAAG CCCTCTATAT AAACAAATAT TGGAGTGAAG
TTGTTGCATA ACTTGCATCG AACAATTAAT AGAAATAACA GAAAATTAAA
AAAGAAATAT G,
```

31

Lbc$_1$ with the sequence

```
TTCTCTTAAT ACAATGGAGT TTTTGTTGAA CATACATACA TTTAAAAAAA
AATCTCTAGT GTCTATTTAC CCGGTGAGAA GCCTTCTCGT GTTTTACACA
CTTTAATATT ATTATATCCT CAACCCCACA AAAAAGAATA CTGTTATATC
TTTCCAAACC TGTAGATTTA TTTATTTATT TATTTATTTT TACAAAGGAG
ACTTCAGAAA AGTAATTACA TAAAGATAGT GAACATCATT TTATTTATTA
TAATAAACTT TAAAATCAAA CTTTTTTATA TTTTTTGTTA CCCTTTTCAT
TATTGGGTGA AATCTCATAG TGAAGCCATT AAATAATTTG GGCTCAAGTT
TTATTAGTAA AGTCTGCATG AAATTTAACT TAACAATAGA GAGAGTTTTC
GAAAGGGAGC GAATGTTAAA AAGTGTGATA TTATATTTTA TTTCGATTAA
TAATTATGTT TACATGAAAA CATACAAAAA AATACTTTTA AATTCAGAAT
AATACTTAAA ATATTTATTT GCTTAATTGA TTAACTGAAA ATTATTTGAT
TAGGATTTTG AAAAGATCAT TGGCTCTTCG TCATGCCGAT TGACACCCTC
CACAAGCCAA GAGAAACTTA AGTTGTAAAC TTTCTCACTC CAAGCCTTCT
ATATAAACAT GTATTGGATG TGAAGTTATT GCATAACTTG CATTGAACAA
TAGAAAATAA CAAAAAAAG TAAAAAGTA GAAAGAAAT ATG,
```

Lbc$_2$ with the sequence:

```
TCGAGTTTTT ACTGAACATA CATTTATTAA AAAAAACTCT CTAGTGTCCA
TTTATTCGGC GAGAAGCCTT CTCGTGCTTT ACACACTTTA ATATTATTAT
ATCCCCACCC CCACCAAAAA AAAAAAAACT GTTATATCTT TCCAGTACAT
TTATTTCTTA TTTTTACAAA GGAAACTTCA CGAAAGTAAT TACAAAAAAG
ATAGTGAACA TCATTTTTTT AGTTAAGATG AATTTTAAAA TCACACTTTT
TTATATTTTT TTGTTACCCT TTTCATTATT GGGTGAAATC TCATAGTGAA
ACTATTAAAT AGTTTGGGCT CAAGTTTTAT TAGTAAAGTC TGCATGAAAT
TTAACTTAAT AATAGAGAGA GTTTTGGAAA GGTAACGAAT GTTAGAAAGT
GTGATATTAT TATAGTTTTA TTTAGATTAA TAATTATGTT TACATGAAAA
TTGACAATTT ATTTTTAAAA TTCAGAGTAA TACTTAAATT ACTTATTTAC
TTTAAGATTT TGAAAGATC ATTTGGCTCT TCATCATGCC GATTGACACC
CTCCACAAGC CAAGAGAAAC TTAAGTTGTA ATTTTTCTAA CTCCAAGCCT
TCTATATAAA CACGTATTGG ATGTGAAGTT GTTGCATAAC TTGCATTGAA
CAATAGAAAT AACAACAAAG AAAATAAGTG AAAAAAGAAA TATG,
```

and Lbc$_3$ with the sequence:

```
TATGAAGATT AAAAAATACA CTCATATATA TGCCATAAGA ACCAACAAAA
GTACTATTTA AGAAAGAAA AAAAAAACCT GCTACATAAT TTCCAATCTT
GTAGATTTAT TTCTTTTATT TTTATAAAGG AGAGTTAAAA AAATTACAAA
ATAAAAATAG TGAACATCGT CTAAGCATTT TTATATAAGA TGAATTTTAA
AAATATAATT TTTTTGTCTA AATCGTATGT ATCTTGTCTT AGAGCCATTT
TTGTTTAAAT TGGATAAGAT CACACTATAA AGTTCTTCCT CCGAGTTTGA
TATAAAAAAA ATTGTTTCCC TTTTGATTAT TGGATAAAAT CTCGTAGTGA
CATTATATTA AAAAAATTAG GGCTCAATTT TTATTAGTAT AGTTTGCATA
AATTTTAACT TAAAAATAGA GAAAATCTGG AAAAGGGACT GTTAAAAAGT
GTGATATTAG AAATTTGTCG GATATATTAA TATTTTATTT TATATGGAAA
CTAAAAAAAT ATATATTAAA ATTTTAAATT CAGAATAATA CTTAAATTAT
TTATTTACTG AAAATGAGTT GATTTAAGTT TTTGAAAAGA TGATTGTCTC
TTCACCATAC CAATTGATCA CCCTCCTCCA ACAAGCCAAG AGAGACATAA
GTTTTATTAG TTATTCTGAT CACTCTTCAA GCCTTCTATA TAAATAAGTA
TTGGATGTGA AGTTGTTGCA TAACTTGCAT TGAACAATTA ATAGAAATAA
CAGAAAAGTA GAAAGAAAT ATG.
```

Another example of a preferred DNA fragment according to the invention is a DNA fragment which is identical with, derived from or comprises 5' flanking regions of the Lbc$_3$-5'-3'CAT gene with the sequence

```
TATGAAGATT AAAAAATACA CTCATATATA TGCCATAAGA ACCAACAAAA
GTACTATTTA AGAAAGAAA AAAAAAACCT GCTACATAAT TTCCAATCTT
GTAGATTTAT TTCTTTTATT TTTATAAAGG AGAGTTAAAA AAATTACAAA
ATAAAAATAG TGAACATCGT CTAAGCATTT TTATATAAGA TGAATTTTAA
AAATATAATT TTTTTGTCTA AATCGTATGT ATCTTGTCTT AGAGCCATTT
TTGTTTAAAT TGGATAAGAT CACACTATAA AGTTCTTCCT CCGAGTTTGA
TATAAAAAAA ATTGTTTCCC TTTTGATTAT TGGATAAAAT CTCGTAGTGA
CATTATATTA AAAAAATTAG GGCTCAATTT TTATTAGTAT AGTTTGCATA
AATTTTAACT TAAAAATAGA GAAAATCTGG AAAAGGGACT GTTAAAAAGT
GTGATATTAG AAATTTGTCG GATATATTAA TATTTTATTT TATATGGAAA
CTAAAAAAAT ATATATTAAA ATTTTAAATT CAGAATAATA CTTAAATTAT
TTATTTACTG AAAATGAGTT GATTTAAGTT TTTGAAAAGA TGATTGTCTC
TTCACCATAC CAATTGATCA CCCTCCTCCA ACAAGCCAAG AGAGACATAA
GTTTTATTAG TTATTCTGAT CACTCTTCAA GCCTTCTATA TAAATAAGTA
TTGGATGTGA AGTTGTTGCA TAACTTGCAT TGAACAATTA ATAGAAATAA
CAGAAAAGTA GAATTCTAAA ATG
```

Still another example of such a DNA fragment ac-

33

cording to the invention is a DNA fragment which is identical with, derived from or comprises 5' flanking regions of the N23 gene with the sequence

```
          10        20        30        40        50        60        70
GAATTCGAGCTCGCCCGGGGATCGATCCTCTAGAGTCGACCTGCAGCCCAAGCTTGGATCAATCAATTAA
EcoRI                             SalI

          80        90       100       110       120       130       140
TTCTATTGAGACACGATTTGAACAATTTTTACATTATGAGACTATTTTTGGTTTTTTATTTGATCCAAAA

         150       160       170       180       190       200       210
AAATTTAAAGCTTTAGATGATGATGAATTGAANNAATATATTGTATTAATNNTGAAAAGTTNNNNNGGTTTA

         220       230       240       250       260       270       280
ATGAATGCTATGATATTGATGGTCTTGATNTATTNNCAGAATTGAAAGTATTAAGAGAAGTGTTAAGAAA

         290       300       310       320       330       340       350
AGAAGTTAGCACACCAATAGAAGTATTGAGTTATATTAAAACTTTAGATTCTTTTCAAATGTTTACATTG

         360       370       380       390       400       410       420
CATATAGAATTTTATTGACAATCCTTATAACAGTTGCTACTGTTGAAAGACGTTCTTCAAAATTAAAATT

         430       440       450       460       470       480       490
ACTTAAATCATATCTAAAATCAACAATGTTACAAGATAGATTGAATGAGTTAGTTATTTTATCTATTGAA

         500       510       520       530       540       550       560
AGTAAAGTGTTAGAATTGTTTGATTATAAAACTCTGATAAATGATTTTGCAGTTAAAAAAACTAGAAGAT

         570       580       590       600       610       620       630
TAATATAAAAATTGATATTTTATATAATATATTAAGTCTCTTTAAAATTCTTGTAAAAAAAGACATTTTT

         640       650       660       670       680       690       700
AAATAATAAAATAAAGCAACTCTTAATTTTAATGAAACATCCCTTTGTTAAACCGAATCTTCCATAATGT

         710       720       730       740       750       760       770
AAAAATTAATGCTTGATGGAAGTTTTTAATTTGTTCTACTCAATACTCAAAGGGTTGTAAATATTTTTTT

         780       790       800       810       820       830       840
TATCATTTATATGTTGTAAATATGAATGCACTAGTAATTAGTTTAATGATAAAATATATTCTACAGATAT

         850       860       870       880       890       900       910
ATTTCTGTCTCTTGGCAACTCGTGAGAATTGAATATATTATAAAGATGAAAGGTCGTTACAATTTTTTTT

         920       930       940       950       960       970       980
AGAATAAATATTTATATACAATTCCTAGATTTTGTTATAAAAATTCACATATTGTATGAGTATAAATACAT

         990      1000      1010      1020      1030      1040      1050
GAGCACACACCAAACTAGTCTCAAATTAAGTAAGGTGCTAATTATTAGCGGCTAGCTAAGTAACCAAGTA
                                                          DdeI

ATTAATG
```

34

The invention relates furthermore to any plasmid to be used when carrying out the method according to the invention and characterised by comprising a DNA fragment containing an inducible plant promoter as herein defined. Particular examples of suitable plasmids according to the invention are pAR11, pAR29, pAR30, and N23-CAT, cf. Examples 3, 4, and 11. These plasmids allow recombination into the A. rhizogenes T DNA region.

The invention relates furthermore to any Agrobacterium strain to be used in connection with the invention and characterised by comprising a DNA fragment comprising an inducible plant promoter of root nodule-specific genes built into the T DNA region and therefore capable of transforming the inducible promoter into plants. Particular examples of bacterium strains according to the invention are the A. rhizogenes strains AR1127 carrying pAR29, AR1134 carrying pAR30, AR1000 carrying pAR11, and AR204-N23-CAT carrying N23-CAT.

It is obvious that the patent protection of the present invention is not limited by the embodiments stated above.

Thus the invention employs not exclusively 5' flanking regions of soybean leghemoglobin genes. It is well-known that the leghemoglobin genes of all leguminous plants have the same function, cf. Appleby (1974) in The Biology of Nitrogen Fixation, Quispel. A. Ed. North-Holland Publishing Company, Amsterdam, Oxford, pages 499-554, and concerning the kidney bean PvLb1 gene it has furthermore been

35

proved that a high degree of homogoly exists with the sequences of the soybean $Lbc_3$ gene. It is also known that the expression of other root nodule-specific genes is regulated in a similar manner like the leghemoglobin genes. The invention includes thus the use of 5' flanking regions of leghemoglobin genes or other root nodule-specific genes of all plants in case the use of such DNA fragments makes the expression of a desired gene product the subject matter of the regulation characterised by the present invention.

The present invention allows also the use of such fragments of any origin which under natural conditions exert or mediate the regulation characterised by the present invention. The latter applies especially to such fragments which can be isolated from DNA fragments from gene libraries or genomes through hybridization with labelled sequences of 5' flanking regions of soybean leghemoglobin genes.

It is well-known that it is possible to alter nucleotide sequences of non-important sub-regions of 5' flanking regions without causing an alteration of the promoter activity and the regulation. It is also well-known that an alteration of sequences of important subregions of 5' flanking regions renders it possible to alter the binding affinities between nucleotide sequences and the factors or effector substances necessary or responsible for the transcription initation and the translation initiation and consequently to improve the promoter activity and/or the regulation. The present invention includes, of course, also the use of DNA fragments

36

containing such altered sequences of 5'flanking regions, and in particular DNA fragments can be mentioned which have been produced by recombining sequences of 5' flanking regions of any gene with 5' flanking regions of root nodule-specific genes provided the use of such DNA fragments subjects the expression of a desired gene product to the regulation characterised by the present invention.

It should be noted that the transformation of microorganisms is carried out in a manner known per se, cf. e.g. Maniatis et al., (1982), Molecular Cloning, A Laboratory Manual, Cold Spring Harbor Laboratory.

The transformation of plant cells, i.e. introduction of plasmid DNA into plant cells, is also carried out in a manner known per se, cf. Zambryski et al., (1983), EMBO J. 2, 2143-2150.

Cleavage with restriction endonucleases and digestion with other DNA modifying enzymes are well-known techniques and are carried out as recommended by the suppliers.

The Agrobacterium rhizogenes 15834 rif$^R$ was used as a typical representative of A. rhizogenes: see White et al., I.Bact., Vol. 141 (1980), 1134-1141.

Example 1

Sequence determination of 5' flanking regions of soybean leghemoglobin genes

From a soybean gene library the four soybean leg-

hemoglobin genes Lba, $Lbc_1$, $Lbc_2$, and $Lbc_3$ are provided as described by Jensen, E.Ø. et al., Nature Vol. 291, No. 3817, 677-679 (1981). The genetically stable in-bred invariable soybean species "Glycine max.var.Evans" was used as a starting material for the isolation of the DNA used for the construction of said gene library. The 5' flanking regions of the four soybean leghemoglobin genes are isolated, as described by Jensen, E.Ø., Ph D Thesis, Institut for Molekylær Biologi, Århus Universitet (1985), and the DNA sequences determined by the use of the dideoxy method as described by Sanger, F., J. Mol. Bio. 143, 161-178 (1980) and indicated in the sequence scheme.

## Example 2

## Construction of $Lbc_3$-5'-3'-CAT

The construction has been carried out in a sequence of process steps as described below:

a)    Sub-cloning the $Lbc_3$ gene

The $Lbc_3$ gene was isolated on a 12Kb EcoRI restriction fragment from a soybean DNA library, which has been described by Wiborg et al., in Nucl. Acids Res. (1982) 10, 3487. A section of the fragment is shown at the top of the attached Scheme 2. This fragment was digested by the enzymes stated and then ligated to pBR322 as indicated at the Scheme. The resulting plasmids $Lbc_3HH$ and $Lbc_3HX$ were subsequently digested by PvuII and religated, which resulted in two plasmids called pLpHH and pLpHX.

b)    Sub-cloning 5' flanking sequences from the Lbc₃ gene

For this purpose pLpHH was used as shown in the attached Scheme 3. This plasmid was opened by means of PvuII and treated with exonuclease Bal31. The reaction was stopped at various times and the shortened plasmids were ligated into fragments from pBR322. These fragments had been treated in advance as shown in Scheme 3, in such a manner that in one end they had a DNA sequence $\begin{matrix} TTC \ --- \\ AAG \ --- \end{matrix}$ .

After the ligation a digestion with EcoRI took place, and the fragments containing 5' flanking sequences were ligated into EcoRI digested pBR322. These plasmids were transformed into E. coli K803, and the plasmids in the transformants were tested by sequence analysis. A plasmid, p213 5'Lb, isolated from one of the transformants, contained a 5' flanking sequence terminating 7 bp before the Lb ATG start codon in such a manner that the sequence is as follows:

                2Kb
-5' flanking --- AAAGTAGAATTC
Lbc₃ sequence

E.coli K803 is a typical representative of the E. coli K12 recipient strains.

c)    Sub-cloning 3' flanking region of the Lbc₃ gene

For this purpose pLpHX was used which was digested by XhoII. The ends were partially filled out and excess single-stranded DNA was removed with S1 nuclease, as shown in the attached Scheme 4. The fragment shown was ligated into pBR322 which had been pretreated as shown in the Scheme. The construction was transformed into E. coli K803. One of the transformants contained a plasmid called Xho2a-3'Lb. As the XhoII recognition sequence is positioned immediately after the Lb stop codon, cf. Scheme 2, the plasmid contained about 900 bp of the 3' flanking region, and the sequence started with GAATTCTACAA----.

The construction of Lb promoter cassette

An EcoRI/SphI fragment from Xho2a-3'Lb was mixed with a BamHI/EcoRI fragment from p213-5'Lb. These two fragments were ligated via the BamHI/SphI cleavage sites into a pBR322 derivative where the EcoRI recognition sequence had been removed, cf. Scheme 4. The ligated plasmids were transformed into E. coli K803. A plasmid in one of the transformants contained the correct fragments, and it was called pEJLb 5'-3'-1.

Construction of the Lbc_3 5'3'-CAT gene

The CAT gene of pBR322 was isolated on several smaller restriction fragments, as shown in the attached Scheme 5. The 5' coding region was isolated as an AluI fragment which was subsequently ligated into pBR322, treated as stated in the Scheme. This

40

was transformed into E. coli K803. Several transformants contained the correct plasmid. One was
taken, out and called Alull. The 3' coding region
was isolated on a TaqI fragment. This fragment was
treated with exonuclease Bal31, whereafter EcoRI
linkers were added. Then followed a digestion with
EcoRI and a ligation to EcoRI digested pBR322. The
latter was transformed into E. coli K803 and the
transformants were analysed. A plasmid, Taq 12,
contained the 3' coding region of the CAT gene
plus 23 bp 3' flanking sequences subsequently terminating in the following sequence CCCCGAATTC.
Subsequently the following fragments were ligated
together to EcoRI digested
pEJLb5'-3'-1: EcoRI/PvuII fragment from AluI,
PvuII/DdeI fragment from pBR322 and DdeI/EcoRI
fragment from Taq 12. This ligation mixture was
transformed into E. coli K803. Several transformants
contained the correct plasmid. One was taken out
and was called pEJLb 5'-3' CAT 15.


Example 3


a.


Cloning and integration of the soybean Lbc$_3$-5'-3'-
CAT gene.


Two EcoRI fragments (No. 36 and No. 40) of the T$_L$-
DNA region of A. rhizogenes 15834 pRi plasmid was
used as "integration sites". Thus the Lbc$_3$-5'-3-
CAT gene was subcloned (as 3,6 Kb BamHI/SalI fragment) into two vectors pAR1 and pAR22 carrying the
above EcoRI fragments. The resulting plasmids pAR29

41

and pAR30 were separately mobilized into <u>A. rhizogenes</u> 15834 rif[R] using a plasmid helper system; see E. van Haute et al. (1983), EMBO J. 3, 411-417. Neither pAR29 nor pAR30 can replicate in Agrobacterium. Therefore the selection by means of rifampicin 100 $\mu$g/ml and the plasmid markers spectinomycine 100 $\mu$g/ml, streptomycine 100 $\mu$g/ml or kanamycine 300 $\mu$g/ml will select <u>A. rhizogenes</u> bacteria having integrated the plasmids via homologous recombination through the EcoRI fragments 36 or 40. The structure of the resulting $T_L$-DNA regions - transferred to the transformed plant lines L5-9 and L6-23 - has been indicated at the bottom of the attached Scheme 6. In this Scheme is furthermore for the L6-23 line shown the EcoRI and HindIII fragments carrying the $Lbc_3$-5'-3'-CAT gene and therefore hybridizing to radioactively labelled $Lbc_3$-5'-3'-CAT DNA used as a probe, cf. Example 4<u>a</u>.

b.

<u>Cloning and integration of the soybean $Lbc_3$ gene.</u>

The EcoRI fragment No. 40 has here been used as "integration site". The $Lbc_3$ gene was therefore sub-cloned (as a 3,6 Kb BamHI fragment into the pAR1 vector and transferred into the $T_L$-DNA region as stated in a. The structure of the $T_L$-DNA region, transferred to the transformed plant line L8-35, has been shown at the bottom of the attached Scheme 7. This Scheme furthermore shows the EcoRI and HindIII fragments carrying the $Lbc_3$ gene and there-

42

fore hybridizing with radioactively labelled Lbc$_3$
DNA used as a probe, cf. Example 4b.

43

## Example 4.

### a.

### Demonstration of the soybean $Lbc_3$-5'-3'-CAT gene in transformed plants of bird's-foot trefoil.

44

DNA extracted from transformed lines (L6-23) or untransformed control plants and cleaved by the restriction enzymes EcoRI and HindIII was analyzed by Southern-hybridization. Radioactively labelled $Lbc_3$-5'-3'-CAT gene was used as a probe for demonstrating corresponding sequences in the transformed lines. The bands marked with numbers correspond to restriction fragments constituting parts of the $Lbc_3$-5'-3'-CAT gene as stated in the restriction map (Scheme 6) of Example 3a.

b.

Demonstration of the soybean $Lbc_3$ gene of transformed plants of bird's-foot trefoil.

DNA extracted from transformed lines (L8-35) or untransformed control plants and cleaved by the restriction enzymes EcoRI and HindIII was analyzed by Southern-hybridization. Radioactive $Lbc_3$ gene was used as a probe for detecting corresponding sequences in the transformed lines. The bands marked with numbers correspond to restriction fragments constituting parts of the $Lbc_3$ gene as stated in the restriction map (Scheme 7) of Example 3b.

46

Example 5

a.

Expression of the $Lbc_3$-5'-3'-CAT gene in various tissues of bird's-foot trefoil.

47

The activity of the chloroamphenicol acetyl transferase (CAT) enzyme is measured as the amount of acetylated chloroamphenicol (AcCm) produced from $^{14}C$-chloroamphenicol. In (a) the acetylated forms 1AcCm and 3AcCm appear, which have been separated from Cm through thin-layer chromatography in chloroform/methanol (95:5). The columns 1-3 show that no CAT activity occurs in root (R), nodule (N), as well as leaves + stem (LS) of untransformed plants of bird's-foot trefoil. The columns 4-6 and 7-9 show the CAT activity in corresponding tissues of $Lbc_3$-5'-3'-CAT transformed L6-23 and L5-9 plants. The conversion of chloroamphenicol in columns 5 and 8 shows the organ-specific expression of the $Lbc_3$-5'-3'-CAT gene in root nodules. The columns 10-12 show the lack of CAT activity in plants transformed with the $Lbc_3$ gene.

b.

Table

| | L6-23 CAT activity | L5-9 CAT activity |
|---|---|---|
| Root | 0 | 0 |
| Nodule | 68830 cpm/µg protein·h | 154,000 cpm/µg protein·h |
| Leaves + Stem | 0 | 0 |

In the Table (b) the CAT activity in $Lbc_3$-5'-3'-CAT transformed L5-9 and L6-23 plants has been stated as the amount of $^{14}C$-chloroamphenicol converted into acetylated derivatives. The amount of radioactivity in the acetylated derivatives has been

48

counted by liquid scintillation and stated in cpm/µg
protein · hour.

Example 6

Transcription test (Northern analysis) on tissues
of Lbc$_3$-5'-3'-CAT transformed and Lbc$_3$ transformed
Lotus plant lines.

49

5 μg of total RNA extracted from root (R), nodule (N) or leaves + stem (LS) and separated in formaldehyde agarose gels were transferred onto nitrocellulose. Column 1 contains 5 μg of total RNA from 20-day-old soybean nodules as control plants. The columns 2-4 and 5-7 contain total RNA from root, nodule or leaves + stem, respectively, of the $Lbc_3$-5'-3'-CAT transformed lines L5-9 and L6-23. The columns 8-10 contain RNA from corresponding tissues of bird's-foot trefoil transformed by means of <u>A. rhizogenes</u> carrying the $Lbc_3$ gene in the $T_L$-DNA. In (a) radioactive DNA of the CAT coding sequence has been used as a probe for hybridization. The organ-specific transcription of the $Lbc_3$-5'-3'-CAT gene in root nodules from the L5-9 and L6-23 lines appears from columns 3 and 6. In (b) the transcript for the constitutive ubiquitine gene(s) is visualized using a cDNA probe for the human ubiquitine gene for the hybridization. In (c) the nodule-specific transcription of bird's-foot trefoil <u>own</u> leghemoglobin genes is shown. A cDNA probe of the Lba gene of soybean has been used for this hybridization.

50

## Example 7

### Determination of the transcription initiation site (CAP site) of the Lbc₃ promoter of soybean in transformed root nodules of bird's-foot trefoil.

Size marker

polyA + mRNA    polyA + mRNA    polyA + mRNA
L6-23           L5-9            Control
N    LS    N

85-

—TATAAATAAGTATTGGATGTGAAGTTGTTGCₙTAACT--/ /—AAATGGAG

83

The position of the "CAP site" was determined on the nucleotide level by means of primer extension. A synthetic oligonucleotide 5'CAACGGTGGTATATCCAGTG3' complementary to the nucleotides 15-34 in the coding sequence of the CAT gene was used as primer for the enzyme reverse transcriptase. As a result single-stranded cDNA was formed the length of which corresponds to the distance between the 5' end of the primer and the 5' end of the primed mRNA. A 83 nucleotide cDNA strand would be expected according to the knowledge of the transcription initiation site of soybean $Lbc_3$ gene. Columns 2, 3, and 4 from left to right show the produced DNA strands when the primer extension has been operated on $polyA^+$-purified mRNA from transformed root nodules of bird's-foot trefoil, transformed leaves + stem of bird's-foot trefoil, and untransformed root nodules of bird's-foot trefoil, respectively. The 85, 86, 87, 88, and 90 nucleotides long cDNA strand shown in column 2 proved correctly $Lbc_3$ promoter function in bird's-foot trefoil. The CAP sites corresponding to the cDNA sequences generated are indicated with asterisks (*) on the partial sequence of the $Lbc_3$ 5'3'-CAT region given. In the sequence the TATA box of the $Lbc_3$ promoter and the corresponding translation initiation codon of the CAT coding sequence are underlined.

52

Example 8

Demonstration of the correct developmental control
of the Lbc$_3$-5'-3'-CAT gene in transformed plants of
bird's-foot trefoil (L6-23).

| | Stage 1: No visible nodules | Stage 2: Emerging nodules | Stage 3: Distinct white nodules | Stage 4: Small pink nodules | Stage 5: Later stages of maturity |
|---|---|---|---|---|---|
| CAT activity in cpm/$\mu$g protein·hour | 0 | 0 | 32.6 | 342.3 | 1255* |
| Nitrogenase activity nmol ethylene/$\mu$g protein · hour | 0 | O | 0 | O.5 | 2.7 |

* Substrate limited reaction; actual activity about
68000 cpm/$\mu$g protein · hour.

Chloroamphenicol acetyl transferase and nitrogenase
activity were measured on cut off pieces of root
with nodules at the different developmental stages
indicated. The CAT activity can be detected in the
white distinct nodules whereas the nitrogenase
activity did not appear until the small pink nodules
have developed. The latter development corresponds
to the development known from soybean control plants
and described by Marcker et al. EMBO J. 1984, 3,
1691-95. The CAT activity was determined as in
Example 5. The nitrogenase activity was measured

53

as acetylene reduction capacity of the nodules followed by gaschromatographic determination of ethylene.

Example 9

Demonstration of $Lbc_3$ protein in bird's-foot trefoil plants transformed with the soybean $Lbc_3$ gene.

Proteins extracted from root nodules of $Lbc_3$ transformed (L8-35), $Lbc_3$-5'-3'-CAT transformed and nontransformed plants were separated by isolectric focussing at a pH gradient of 4 to 5. The columns 1, 3, 5, 7, and 9 show $Lbc_1$, $Lbc_2$, $Lbc_3$, and Lba proteins synthesized in soybean control root nodules. Column 2 shows proteins from root nodules of $Lbc_3$-5'-3'-CAT transformed L6-23-bird's-foot trefoil plants, whereas the columns 6 and 8 show proteins from nontransformed plants. The columns 4 and 10 show soybean $Lbc_3$ protein synthesized in root nod-

54

ules of bird's-foot trefoil plants (L8-35) trans-
formed with the Lbc$_3$ gene. The Lbc$_3$ protein band
is indicated by an arrow.

Example 10

Expression of the Lbc$_3$-5'-3'-CAT gene requires the
5' Lbc$_3$ promoter region.

The Lbc$_3$-5'-3'-CAT gene construction carries a 2 Kb ·
5' Lbc$_3$ promoter region. Stepwise removal of sequences from the 5' end of this region demonstrated
that this promoter region is required for the char-
acteristic expression of the Lbc$_3$-5'3'-CAT gene.

The Lbc$_3$-5'-3'-CAT gene construction was opened in
the unique XbaI site shown above, and digested with
the exonuclease Bal31. A SalI linker fragment was
ligated onto the blunt ends generated and the shortened SalI fragments carrying the Lbc$_3$-5'-3'-CAT gene
were transferred into L.corniculatus. The effect
of removing promoter sequences was measured as CAT
activity. End points of the deleted 5' region are
given as the distance from the CAP site in nucleo-
tides.

55

|  | | | CAT activity Cpm/µg protein/hrs. | | |
|---|---|---|---|---|---|
| 5'Lbc$_3$ | | 3'Lbc$_3$ | Root | Nodule | Leaf |
| 2000 CAT | | | 0 | 80000 | 0 |
| -950 | | | 0 | 10000 | 0 |
| -474 | | | 0 | 3000 | 0 |
| -230 | | | 0 | 3000 | 0 |
| -78 | | | 0 | 0 | 0 |

The drastically reduced level of CAT activity expressed from the Lbc$_3$ promoter deleted to nucleotide -230 and the zero activity from the promoter deleted to nucleotide -78 demonstrates that the Lbc$_3$ promoter region is required for the root nodule specific expression of the Lbc$_3$-5'-3'-CAT gene.

Example 11

Construction of the N23-CAT gene.

The N23 gene was isolated from a soybean DNA library as described in the enclosed paper of Sandal, Bojsen and Marcker. The N23-CAT gene was constructed from the modified Lbc$_3$-5'-3'-CAT gene carried on plasmid pEJ5'-3'-CAT101 as described in the Applicant's copending application No. 86 11 4704.9 concerning "Expression of Genes in Yeast", and a 1 Kb. EcoRI, DdeI fragment containing the N23 5' promoter region. The position of the EcoRI and DdeI sites in the N23 promoter region is indicated on the DNA sequence shown below. The cloning procedure used is outlined

56

below. The disclosure of the papers of Sandal et al., the EP application, and the paper of Jensen et al., Nature 321 (12 June 1986), 669-674, including the references cited should be considered incorporated into the present description as a means to amend, illustrate, and clarify it.

The N23-CAT gene was transferred to plants by the same method as the Lbc$_3$-5'-3'-CAT gene.

57

0249676

Not to scale

58

DNA sequence of the 5'-promotor region from the N23 gene


```
          10        20        30        40        50        60        70
GAATTCGAGCTCGCCCGGGGATCGATCCTCTAGAGTCGACCTGCAGCCCAAGCTTGGATCAATCAATTAA
EcoRI                              SalI

          80        90       100       110       120       130       140
TTCTATTGAGACACGATTTGAACAATTTTTACATTATGAGACTATTTTTGGTTTTTTATTTGATCCAAAA

         150       160       170       180       190       200       210
AAATTTAAAGCTTTAGATGATGATGAATTGAANNAATATTGTATTAATNNTGAAAAGTTNNNNNGGTTTA

         220       230       240       250       260       270       280
ATGAATGCTATGATATTGATGGTCTTGATNTATTNNCAGAATTGAAAGTATTAAGAGAAGTGTTAAGAAA

         290       300       310       320       330       340       350
AGAAGTTAGCACACCAATAGAAGTATTGAGTTATATTAAAACTTTAGATTCTTTTCAAATGTTTACATTG

         360       370       380       390       400       410       420
CATATAGAATTTTATTGACAATCCTTATAACAGTTGCTACTGTTGAAAGACGTTCTTCAAAATTAAAATT

         430       440       450       460       470       480       490
ACTTAAATCATATCTAAAATCAACAATGTTACAAGATAGATTGAATGAGTTAGTTATTTTATCTATTGAA

         500       510       520       530       540       550       560
AGTAAAGTGTTAGAATTGTTTGATTATAAAACTCTGATAAATGATTTTGCAGTTAAAAAAACTAGAAGAT

         570       580       590       600       610       620       630
TAATATAAAAATTGATATTTTATATAATATATTAAGTCTCTTTAAAATTCTTGTAAAAAAAGACATTTTT

         640       650       660       670       680       690       700
AAATAATAAAATAAAGCAACTCTTAATTTTAATGAAACATCCCTTTGTTAAACCGAATCTTCCATAATGT

         710       720       730       740       750       760       770
AAAAATTAATGCTTGATGGAAGTTTTTAATTTGTTCTACTCAATACTCAAAGGGTTGTAAATATTTTTTT

         780       790       800       810       820       830       840
TATCATTTATATGTTGTAAATATGAATGCACTAGTAATTAGTTTAATGATAAAATATATTCTACAGATAT

         850       860       870       880       890       900       910
ATTTCTGTCTCTTGGCAACTCGTGAGAATTGAATATATTATAAAGATGAAAGGTCGTTACAATTTTTTTT

         920       930       940       950       960       970       980
AGAATAAATATTTATATACAATTCCTAGATTTTGTTATAAAATTCACATATTGTATGAGTATAAATACAT

         990      1000      1010      1020      1030      1040      1050
GAGCACACACCAAACTAGTCTCAAATTAAGTAAGGTGCTAATTATTAGCGGCTAGCTAAGTAACCAAGTA
                                                            DdeI
```

ATTAATG

59

Example 12

<u>Organ-specific expression of the soybean N23-CAT gene in root nodules of L.corniculatus and Trifolium repens.</u>

The activity of chloroamphenicol acetyl transferase (CAT) was measured as in example 5 and is given in cpm/$\mu$g protein/hrs.

| <u>Table a.</u> | | CAT activity |
|---|---|---|
| N23-CAT transformed | | Untransformed |
| <u>L.corniculatus</u> | | <u>L.corniculatus</u> |
| Root nodule | 86150 | 0 |
| Root | 0 | 0 |

| <u>Table b.</u> | | CAT activity |
|---|---|---|
| N23-CAT transformed | | Untransformed |
| <u>T.repens</u> | | <u>T.repens</u> |
| Root nodule | 148000 | 0 |
| Root | 0 | 0 |

Table (a) and b) shows the organ-specific expression of the N23-CAT gene in root nodules of <u>L.corniculatus</u> and <u>T.repens</u>. <u>L.corniculatus</u> was inoculated with <u>Rhizobium loti</u>, while <u>T.repens</u> was inoculated with <u>Rhizobium trifolii</u>.

In connection with the invention it has thus been proved that root nodule-specific genes can be expressed organ-specifically upon transfer to other plants, here <u>Lotus corniculatus</u> and <u>Trifolium re-</u>

60

_pens_. It has furthermore been proved that the 5'
flanking regions comprising the promoter are controlled by the organ-specific regulatory mechanism
as the organ-specific control of the $Lbc_3$-5'-3'-CAT
gene in _Lotus_ _corniculatus_ took place at the trans-
cription level. The $Lbc_3$-5'-3'-CAT gene transferred
was thus only transcribed in root nodules of transformed plants and not in other organs such as roots,
stems, and leaves.

The expression of the $Lbc_3$-5'-3'-CAT gene in root
nodules of transformed plants also followed the
developmental timing known from soybean root nodules. No CAT activity could be detected in roots
or small white root nodules (Example 8). A low
activity was present in the further developed white
distinct nodules, whereas a high activity could be
measured in the small pink nodules and mature nodules developed later on.

The organ-specific expression and the correct developmental expression of transferred root nodule-
specific genes, here exemplified by the $Lbc_3$-5'-3'-
CAT gene, allows as a particular use a functional
expression of root nodule-specific genes also in
other plants beyond leguminous plants. When all
the root nodule-specific plant genes necessary for
the formation of root nodules are transferred from
a leguminous plant to a non-root-nodule-forming
plant species, the correct organ-specific expression proved above allows production of functionally
active, nitrogen-fixing root nodules on this plant
upon infection by _Rhizobium_. In this manner these
plants can grow without the supply of external inorganic or organic nitrogen compounds. Root nodule-specific promoters, here exemplified by the $Lbc_3$ and N23 promoters, must be used in the present case for regulating the expression of the transferred genes.

According to the present invention a root nodule-specific promoter is used for expressing genes. The gene product or function of the gene product improves the function of the root nodule, e.g. by altering the oxygen transport, the metabolism, the nitrogen fixation or the nitrogen absorption.

Root nodules are thus used for the synthesis of biological products improving the plant per se or which can be extracted from the plant later on. A root nodule-specific promoter can be used for expressing a gene. The gene product or compound formed by said gene product constitute the desired product(s).

In connection with the present invention it has furthermore been proved that the soybean $Lbc_3$ leghemoglobin protein per se, i.e. the $Lbc_3$ gene product, is present in a high concentration in root nodules of bird's-foot trefoil plants expressing the $Lbc_3$ code sequence under the control of the $Lbc_3$ promoter. The latter has been proved by cloning the genomic $Lbc_3$ gene of the soybean into the integration vector pAR1, said genomic $Lbc_3$ gene containing the coding sequence, the intervening sequences, and the 5' and 3' flanking sequences. A 3.6 Kb BamHI fragment $Lbc_3HH$, cf. Example 2, was cloned into the pAR1 plasmid and transferred to

62

bird's-foot trefoil as stated previously.

The high level of $Lbc_3$ protein, cf. Example 9, found in transformed root nodules of bird's-foot trefoil and corresponding to the level in soybean root nodules proves an efficient transcription of the $Lbc_3$ promoter and an efficient processing and translation of $Lbc_3$mRNA in bird's-foot trefoil.

The high level of the CAT activity present in transformed root nodules is also a result of an efficient translation of mRNA formed from the chimeric $Lbc_3$ gene. The leader sequence on the $Lbc_3$ gene is decisive for the translation initiation and must determine the final translation efficiency. This efficiency is of importance for an efficient synthesis of gene products in plants or plant cells. An $Lbc_3$ or another leghemoglobin leader sequence can thus be used for increasing the final expression level of a predetermined plant promoter. The construction of a DNA fragment comprising a Lb leader sequence as first sequence and an arbitrary promoter as second sequence is a particular use of the invention when the construction is transferred and expressed in plants.

During nodule development around 30 different plant encoded polypeptides (nodulins) are specifically synthesized. Apart from the leghemoglobins, nodulins include nodule-specific forms of uricase (Bergmann et al (1983) EMBO. J. 2, 2333-2339), glutamine synthetase (Cullimore et al (1984) J.Mol. Appl. Genetics 2, 589-599) and sucrose synthase (Morell and Copeland (1985) Plant. Physiol. 78,

63

149-154). The function of most nodulins are, however, at present unknown.

Many nodulin genes have nevertheless been isolated and characterised during the last five years. These include nodulins from several different legumes. Examples of such isolations and characterisations are widespread in the literature such as (Fuller et al (1983) Proc. Natl. Acad.Sci. 80, 2594-2598), (Sengupta-Gopalan et al (1986) Molec. Gen. Genet. 203, 410-420), (Bisseling et al (1985) in Proceedings of the 6th Int. symp. on Nitrogen Fixation, Martinus Nijhoff Publishers pp 53-59.), and (Gebhardt et al (1986) EMBO.J.5, 1429-1435). All of these genes contain nodule-specific regulatory sequences. Such sequences and in fact entire 5' flanking regions and 3' flanking regions can furthermore be synthesized by automated oligonucleotide synthesis knowing the DNA sequences for the $Lbc_3$ and N23 genes given in this description. Entire nodule-specific genes can also be isolated with known recombinant techniques as described in the above papers and by (Maniatis et al (1982) Molecular cloning. A Laboratory Manual, Cold Spring Harbour Laboratory, New York).

The described method to obtain nodule-specific expression of genes can thus be reconstructed and performed according to the invention by any one skilled in the art of molecular genetics.

The method to obtain nodule-specific expression is not dependent on the A. rhizogenes plant transformation described. Any other plant transformation

system e.g. <u>A. tumefaciens</u> systems, direct gene transfer or microinjection can equally be applied.

The <u>A. rhizogenes</u> system has been used and characterised by a number of scientific groups and is thus well-known from the literature. The characteristics of the system is described in:

Willmitzer et al. (1982), Molec.Gen. Genet. 186, 16-22,

Chilton et al. (1982), Nature 295, 432-434,

Simpson et al. (1986), Plant.Molec.Biol. 6, 493-415,

Tepfer D. (1983), Molecular Genetics of the Bacteria - Plant interaction,

Springer Verlag, Berlin Heidelberg pp 248-258,

White and Nester (1980), J.Bact. 144, 710-720,

Jaynes and Strobel (1981), Int.Rev. of Cytol. Sup. 13, 105-125,

White and Nester (1980), J. Bact. 141, 1134-1141,

Pomponi et al. (1983), Plasmid 10, 119-129, and

65

Slightom et al. (1986), J. Biol. Chem.
261, 108-121.

The latter two publications describe the restriction map and nucleotide sequence of the A. rhizogenes $T_L$-DNA segment used in the transformation system described here. With this information it is possible to anybody skilled in molecular genetics to use and reconstruct the "intermediate vectors" and the A. rhizogenes strains described here.

66

Claims:

1. A method of expressing genes in plants, parts of plants, and plant cell cultures by introducing into a cell thereof a recombinant DNA segment containing both the gene to be expressed and a 5' flanking region comprising a promoter sequence, and optionally a 3' flanking region, and culturing of the transformed cells in a growth medium, c h a r a c t e r i s e d by using as the recombinant DNA segment a DNA fragment comprising an inducible plant promoter (as defined) from root nodule-specific genes.

2. A method as claimed in claim 1, c h a r - a c t e r i s e d by using a DNA fragment comprising an inducible plant promoter (as defined) and being identical with, derived from or comprising 5' flanking regions of root nodule-specific genes.

3. A method as claimed in claim 2, c h a r - a c t e r i s e d by using a DNA fragment comprising an inducible plant promoter (as defined) and being identical with, derived from or comprising 5' flanking regions of root nodule-specific genes, said DNA fragment causing an expression of a gene which is induced in root nodules at specific stages of development and as a step of the symbiosis, whereby nitrogen fixation occurs.

4. A method as claimed in claims 1-3 for the expression of root nodule-specific genes, c h a r a c t e r i s e d by using a DNA fragment comprising an inducible plant promoter (as defined) from root nodule-specific genes.

5.    A method as claimed in claims 1-3 for the expression of genes in leguminous plants, parts of leguminous plants, and leguminous plant cell cultures, c h a r a c t e r i s e d  by using a DNA fragment comprising an inducible plant promoter (as defined) from root nodule-specific genes.

6.    A method as claimed in claims 1-5, c h a r-a c t e r i s e d  by the DNA fragment comprising the inducible plant promoter and being identical with, derived from or comprising 5' flanking regions of leghemoglobin genes.

7.    A method as claimed in claim 6, c h a r - a c t e r i s e d  by the DNA fragment comprising the inducible plant promoter and being identical with, derived from or comprising 5' flanking regions of soybean leghemoglobin genes.

8.    A method as claimed in claim 7, c h a r - a c t e r i s e d  by the DNA fragment comprising the inducible plant promoter and being identical with, derived from or comprising 5' flanking regions of the Lba gene with the sequence

```
GAGATACATT ATAATAATCT CTCTAGTGTC TATTTATTAT TTTATCTGGT
GATATATACC TTCTCGTATA CTGTTATTTT TTCAATCTTG TAGATTACT
TCTTTTATTT TTATAAAAAA GACTTTATTT TTTTAAAAAA AATAAAGTGA
ATTTTGAAAA CATGCTCTTT GACAATTTTC TGTTTCCTTT TTCATCATTG
GGTTAAATCT CATAGTGCCT CTATTCAATA ATTTGGGCTC AATTTAATTA
GTAGAGTCTA CATAAAATTT ACCTTAATAG TAGAGAATAG AGAGTCTTGG
AAAGTTGGTT TTTCTCGAGG AAGAAAGGAA ATGTTAAAAA CTGTGATATT
TTTTTTTTGG ATTAATAGTT ATGTTTATAT GAAAACTGAA AATAAATAAA
CTAACCATAT TAAATTTAGA ACAACACTTC AATTATTTTT TTAATTTGAT
TAATTAAAAA ATTATTTGAT TAAATTTTTT AAAAGATCGT TGTTTCTTCT
TCATCATGCT GATTGACACC CTCCACAAGC CAAGAGAAAC ACATAAGCTT
TGGTTTTCTC ACTCTCCAAG CCCTCTATAT AAACAAATAT TGGAGTGAAG
```

68

TTGTTGCATA ACTTGCATCG AACAATTAAT AGAAATAACA GAAAATTAAA
AAAGAAATAT G.

9.      A method as claimed in claim 7, c h a r -
a c t e r i s e d   by the DNA fragment comprising
the inducible plant promoter and being identical
with, derived from or comprising 5' flanking regions
of the $Lbc_1$ gene with the sequence:

TTCTCTTAAT ACAATGGAGT TTTTGTTGAA CATACATACA TTTAAAAAAA
AATCTCTAGT GTCTATTTAC CCGGTGAGAA GCCTTCTCGT GTTTTACACA
CTTTAATATT ATTATATCCT CAACCCCACA AAAAAGAATA CTGTTATATC
TTTCCAAACC TGTAGATTTA TTTATTTATT TATTTATTTT TACAAAGGAG
ACTTCAGAAA AGTAATTACA TAAAGATAGT GAACATCATT TTATTTATTA
TAATAAACTT TAAAATCAAA CTTTTTTATA TTTTTTGTTA CCCTTTTCAT
TATTGGGTGA AATCTCATAG TGAAGCCATT AAATAATTTG GGCTCAAGTT
TTATTAGTAA AGTCTGCATG AAATTTAACT TAACAATAGA GAGAGTTTTC
GAAAGGGAGC GAATGTTAAA AAGTGTGATA TTATATTTTA TTTCGATTAA
TAATTATGTT TACATGAAAA CATACAAAAA AATACTTTTA AATTCAGAAT
AATACTTAAA ATATTTATTT GCTTAATTGA TTAACTGAAA ATTATTTGAT
TAGGATTTTG AAAAGATCAT TGGCTCTTCG TCATGCCGAT TGACACCCTC
CACAAGCCAA GAGAAACTTA AGTTGTAAAC TTTCTCACTC CAAGCCTTCT
ATATAAACAT GTATTGGATG TGAAGTTATT GCATAACTTG CATTGAACAA
TAGAAAATAA CAAAAAAAG TAAAAAGTA GAAAGAAAT ATG,

10.      A method as claimed in claim 7, c h a r -
a c t e r i s e d   by the DNA fragment comprising
the inducible plant promoter and being identical
with, derived from or comprising 5' flanking regions
of the $Lbc_2$ gene with the sequence:

TCGAGTTTTT ACTGAACATA CATTTATTAA AAAAAACTCT CTAGTGTCCA
TTTATTCGGC GAGAAGCCTT CTCGTGCTTT ACACACTTTA ATATTATTAT
ATCCCCACCC CCACCAAAAA AAAAAAAACT GTTATATCTT TCCAGTACAT
TTATTTCTTA TTTTTACAAA GGAAACTTCA CGAAAGTAAT TACAAAAAAG
ATAGTGAACA TCATTTTTTT AGTTAAGATG AATTTTAAAA TCACACTTTT
TTATATTTTT TTGTTACCCT TTTCATTATT GGGTGAAATC TCATAGTGAA
ACTATTAAAT AGTTTGGGCT CAAGTTTTAT TAGTAAAGTC TGCATGAAAT
TTAACTTAAT AATAGAGAGA GTTTTGGAAA GGTAACGAAT GTTAGAAAGT
GTGATATTAT TATAGTTTTA TTTAGATTAA TAATTATGTT TACATGAAAA
TTGACAATTT ATTTTTAAAA TTCAGAGTAA TACTTAAATT ACTTATTTAC
TTTAAGATTT TGAAAAGATC ATTTGGCTCT TCATCATGCC GATTGACACC
CTCCACAAGC CAAGAGAAAC TTAAGTTGTA ATTTTTCTAA CTCCAAGCCT
TCTATATAAA CACGTATTGG ATGTGAAGTT GTTGCATAAC TTGCATTGAA
CAATAGAAAT AACAACAAAG AAAATAAGTG AAAAAAGAAA TATG,

69

11. A method as claimed in claim 7, c h a r -
a c t e r i s e d by the DNA fragment comprising
the inducible plant promoter and being identical
with, derived from or comprising 5' flanking regions
of the Lbc₃ gene with the sequence:

```
TATGAAGATT AAAAAATACA CTCATATATA TGCCATAAGA ACCAACAAAA
GTACTATTTA AGAAAGAAA AAAAAAACCT GCTACATAAT TTCCAATCTT
GTAGATTTAT TTCTTTTATT TTTATAAAGG AGAGTTAAAA AAATTACAAA
ATAAAAATAG TGAACATCGT CTAAGCATTT TTATATAAGA TGAATTTTAA
AAATATAATT TTTTTGTCTA AATCGTATGT ATCTTGTCTT AGAGCCATTT
TTGTTTAAAT TGGATAAGAT CACACTATAA AGTTCTTCCT CCGAGTTTGA
TATAAAAAAA ATTGTTTCCC TTTTGATTAT TGGATAAAAT CTCGTAGTGA
CATTATATTA AAAAAATTAG GGCTCAATTT TTATTAGTAT AGTTTGCATA
AATTTTAACT TAAAAATAGA GAAAATCTGG AAAAGGGACT GTTAAAAAGT
GTGATATTAG AAATTTGTCG GATATATTAA TATTTTATTT TATATGGAAA
CTAAAAAAAT ATATATTAAA ATTTTAAATT CAGAATAATA CTTAAATTAT
TTATTTACTG AAAATGAGTT GATTTAAGTT TTTGAAAAGA TGATTGTCTC
TTCACCATAC CAATTGATCA CCCTCCTCCA ACAAGCCAAG AGAGACATAA
GTTTTATTAG TTATTCTGAT CACTCTTCAA GCCTTCTATA TAAATAAGTA
TTGGATGTGA AGTTGTTGCA TAACTTGCAT TGAACAATTA ATAGAAATAA
CAGAAAAGTA GAAAAGAAAT ATG.
```

12. A method as claimed in claim 7, c h a r a c-
t e r i s e d by the DNA fragment comprising the
inducible plant promoter and being identical with,
derived from or comprising 5' flanking regions of
the Lbc₃-5'-3'-CAT gene with the sequence:

```
TATGAAGATT AAAAAATACA CTCATATATA TGCCATAAGA ACCAACAAAA
GTACTATTTA AGAAAGAAA AAAAAAACCT GCTACATAAT TTCCAATCTT
GTAGATTTAT TTCTTTTATT TTTATAAAGG AGAGTTAAAA AAATTACAAA
ATAAAAATAG TGAACATCGT CTAAGCATTT TTATATAAGA TGAATTTTAA
AAATATAATT TTTTTGTCTA AATCGTATGT ATCTTGTCTT AGAGCCATTT
TTGTTTAAAT TGGATAAGAT CACACTATAA AGTTCTTCCT CCGAGTTTGA
TATAAAAAAA ATTGTTTCCC TTTTGATTAT TGGATAAAAT CTCGTAGTGA
CATTATATTA AAAAAATTAG GGCTCAATTT TTATTAGTAT AGTTTGCATA
AATTTTAACT TAAAAATAGA GAAAATCTGG AAAAGGGACT GTTAAAAAGT
GTGATATTAG AAATTTGTCG GATATATTAA TATTTTATTT TATATGGAAA
CTAAAAAAAT ATATATTAAA ATTTTAAATT CAGAATAATA CTTAAATTAT
TTATTTACTG AAAATGAGTT GATTTAAGTT TTTGAAAAGA TGATTGTCTC
TTCACCATAC CAATTGATCA CCCTCCTCCA ACAAGCCAAG AGAGACATAA
GTTTTATTAG TTATTCTGAT CACTCTTCAA GCCTTCTATA TAAATAAGTA
TTGGATGTGA AGTTGTTGCA TAACTTGCAT TGAACAATTA ATAGAAATAA
CAGAAAAGTA GAATTCTAAA ATG
```

13. A method as claimed in claim 5, c h a r a c-
t e r i s e d by the DNA fragment comprising the
inducible plant promoter and being identical with,
derived from or comprising 5' flanking regions of
the N23 gene with the sequence:

```
        10        20        30        40        50        60        70
GAATTCGAGCTCGCCCGGGGATCGATCCTCTAGAGTCGACCTGCAGCCCAAGCTTGGATCAATCAATTAA
 EcoRI                             SalI

        80        90       100       110       120       130       140
TTCTATTGAGACACGATTTGAACAATTTTTACATTATGAGACTATTTTTGGTTTTTTATTTGATCCAAAA

       150       160       170       180       190       200       210
AAATTTAAAGCTTTAGATGATGATGAATTGAANNAATATTGTATTAATNNNTGAAAAGTTNNNNNGGTTTA

       220       230       240       250       260       270       280
ATGAATGCTATGATATTGATGGTCTTGATNTATTNNCAGAATTGAAAGTATTAAGAGAAGTGTTAAGAAA

       290       300       310       320       330       340       350
AGAAGTTAGCACACCAATAGAAGTATTGAGTTATATTAAAACTTTAGATTCTTTTCAAATGTTTACATTG

       360       370       380       390       400       410       420
CATATAGAATTTTATTGACAATCCTTATAACAGTTGCTACTGTTGAAAGACGTTCTTCAAAATTAAAATT

       430       440       450       460       470       480       490
ACTTAAATCATATCTAAAATCAACAATGTTACAAGATAGATTGAATGAGTTAGTTATTTTATCTATTGAA

       500       510       520       530       540       550       560
AGTAAAGTGTTAGAATTGTTTGATTATAAAACTCTGATAAATGATTTTGCAGTTAAAAAAACTAGAAGAT

       570       580       590       600       610       620       630
TAATATAAAAATTGATATTTTATATAATATATTAAGTCTCTTTAAAATTCTTGTAAAAAAAGACATTTTT

       640       650       660       670       680       690       700
AAATAATAAAATAAAGCAACTCTTAATTTTAATGAAACATCCCTTTGTTAAACCGAATCTTCCATAATGT

       710       720       730       740       750       760       770
AAAAATTAATGCTTGATGGAAGTTTTTAATTTGTTCTACTCAATACTCAAAGGGTTGTAAATATTTTTTT

       780       790       800       810       820       830       840
TATCATTTATATGTTGTAAATATGAATGCACTAGTAATTAGTTTAATGATAAAATATATTCTACAGATAT

       850       860       870       880       890       900       910
ATTTCTGTCTCTTGGCAACTCGTGAGAATTGAATATATTATAAAGATGAAAGGTCGTTACAATTTTTTTT

       920       930       940       950       960       970       980
AGAATAAATATTTATATACAATTCCTAGATTTTGTTATAAAAATTCACATATTGTATGAGTATAAATACAT

       990      1000      1010      1020      1030      1040      1050
GAGCACACACCAAACTAGTCTCAAAATTAAGTAAGGTGCTAATTATTAGCGGCTAGCTAAGTAACCAAGTA
                                                       DdeI

ATTAATG
```

14. A method as claimed in any of the claims 1-13, c h a r a c t e r i s e d by the 3' flanking region of the genes to be expressed being a 3' flanking region of root nodule-specific genes of any origin.

15. A method as claimed in claim 14, c h a r - a c t e r i s e d by the 3' flanking region being of leghemoglobin genes.

16. A method as claimed in claim 14, c h a r - a c t e r i s e d by the 3' flanking region being of soybean leghemoglobin genes.

17. A method as claimed in claim 16, c h a r - a c t e r i s e d by the 3' flanking region being of the Lba, $Lbc_1$, $Lbc_2$ or $Lbc_3$ gene with the fol- lowing sequences, respectively:

Lba

```
                              1590                                    1620
            TAA TTA GTA TCT ATT GCA GTA AAG TGT AAT AAA TAA ATC TTG

                              1650                                    1680
TTT CAC TAT AAA ACT TGT TAC TAT TAG ACA AGG GCC TGA TAC AAA ATG TTG GTT AAA ATA

                              1710                                    1740
ATG GAA TTA TAT AGT ATT GGA TAA AAA TCT TAA GGT TAA TAT TCT ATA TTT GCG TAG GTT

                              1770                                    1800
TAT GCT TGT GAA TCA TTA TCG GTA TTT TTT TTC CTT TCT GAT AAT TAA TCG GTA AAT TA

                              1830                                    1860
ACA AAT AAG TTC AAA ATG ATT TAT ATG TTT CAA AAT TAT TTT AAC AGC AGG TAA AAT GTT

ATT TGG TAC GAA AGC TAA TTC GTC GA
```

72

Lbc₁

```
                                                              1320
                                TAA/TT AGG ATC TAC TGC ATT GCC GTA

                               1350                            1380
AAG TGT AAT AAA TAA ATC TTG TTT CAA CTA AAA CTT GTT ATT AAA CAA GTT CCC TAT ATA

                               1410                           1440
AAT GTT GTT TAA AAT AAG TAA ATT TCA TTG TAT TGG ATA AAC ACT TTT AAG TTA TAT ATT

                               1470                           1500
TCC ATA TAT TTA CGT TTG TGA ATC ATA ATC GAT ACT TTA TAA AAA TAA ATT CCA AAT AAT

TTA TAC GTT TTA AAA ATT ATT TT
```

Lbc₂

```
                                              TAG/GAT CTA CTA TTG CCG TCA AGT
                                                                        1140

    GTA ATA AAT AAA TTT TGT TTC ACT AAA ACT TGT TAT TAA ACA AGT CCC CGA TAT ATA AAT
                                    1170                            1200
    GTT GGT TAA AAT AAG TAA ATT ATA CGG TAT TGA TAA ACA ATC TTA AGT TTT ATA TAT AGT
                                    1230                            1260

    TCC ATA TAC TAA AGT TTG TGA ATC ATA ATC GA
                                    1290
```

and Lbc₃

```
                            TAG/GAT CTA CAA TTG CCT TAA AGT GTA ATA AAT AAA
                                990                              1020

    TAT TAT TTC ACT AAA ACT TGT TAT TAA ACC AAG TTC TCG ATA TAA ATG TTG GTT AAA CTA
                                    1050                            1080

    AGT AAA TTA TAT GGT ATT GGA TAA ACA ATC TTA AGC TT
                                    1110
```

18.    A method as claimed in claim 1 of preparing a polypeptide by introducing into a cell of a plant, a part of a plant or a plant cell culture a recombinant plasmid, c h a r a c t e r i s e d by using as the recombinant plasmid a plasmid comprising an inducible plant promoter (as defined) of root nodule-specific genes.

73

19.   A DNA fragment comprising an inducible plant promoter (as defined) to be used when carrying out the method as claimed in claims 1-18, c h a r - a c t e r i s e d   by being identical with, derived from or comprising a 5' flanking region of root nodule-specific genes of any origin.

20.   A DNA fragment as claimed in claim 19, c h a r a c t e r i s e d   by being identical with, derived from or comprising a 5' flanking region of plant leghemoglobin genes.

21.   A DNA fragment as claimed in claim 20, c h a r a c t e r i s e d   by being identical with, derived from or comprising a 5' flanking region of soybean leghemoglobin genes.

22.   A DNA fragment as claimed in claim 21, c h a r a c t e r i s e d   by being identical with, derived from or comprising a 5' flanking region of the Lba gene with the sequence:

```
GAGATACATT ATAATAATCT CTCTAGTGTC TATTTATTAT TTTATCTGGT
GATATATACC TTCTCGTATA CTGTTATTTT TTCAATCTTG TAGATTTACT
TCTTTTATTT TTATAAAAAA GACTTTATTT TTTTAAAAAA AATAAAGTGA
ATTTTGAAAA CATGCTCTTT GACAATTTTC TGTTTCCTTT TTCATCATTG
GGTTAAATCT CATAGTGCCT CTATTCAATA ATTTGGGCTC AATTTAATTA
GTAGAGTCTA CATAAAATTT ACCTTAATAG TAGAGAATAG AGAGTCTTGG
AAAGTTGGTT TTTCTCGAGG AAGAAAGGAA ATGTTAAAAA CTGTGATATT
TTTTTTTTGG ATTAATAGTT ATGTTATAT GAAAACTGAA AATAAATAAA
CTAACCATAT TAAATTTAGA ACAACACTTC AATTATTTTT TTAATTTGAT
TAATTAAAAA ATTATTTGAT TAAATTTTTT AAAAGATCGT TGTTTCTTCT
TCATCATGCT GATTGACACC CTCCACAAGC CAAGAGAAAC ACATAAGCTT
TGGTTTTCTC ACTCTCCAAG CCCTCTATAT AAACAAATAT TGGAGTGAAG
TTGTTGCATA ACTTGCATCG AACAATTAAT AGAAATAACA GAAAATTAAA
AAAGAAATAT G,
```

74

23. A DNA fragment as claimed in claim 21, c h a r a c t e r i s e d  by being identical with, derived from or comprising a 5' flanking region of the Lbc$_1$ gene with the sequence:

```
  TTCTCTTAAT ACAATGGAGT TTTTGTTGAA CATACATACA TTTAAAAAAA
  AATCTCTAGT GTCTATTTAC CCGGTGAGAA GCCTTCTCGT GTTTTACACA
  CTTTAATATT ATTATATCCT CAACCCCACA AAAAAGAATA CTGTTATATC
  TTTCCAAACC TGTAGATTTA TTTATTTATT TATTTATTTT TACAAAGGAG
  ACTTCAGAAA AGTAATTACA TAAAGATAGT GAACATCATT TTATTTATTA
  TAATAAACTT TAAAATCAAA CTTTTTTATA TTTTTTGTTA CCCTTTTCAT
  TATTGGGTGA AATCTCATAG TGAAGCCATT AAATAATTTG GGCTCAAGTT
  TTATTAGTAA AGTCTGCATG AAATTTAACT TAACAATAGA GAGAGTTTTC
  GAAAGGGAGC GAATGTTAAA AAGTGTGATA TTATATTTTA TTTCGATTAA
  TAATTATGTT TACATGAAAA CATACAAAAA AATACTTTTA AATTCAGAAT
  AATACTTAAA ATATTTATTT GCTTAATTGA TTAACTGAAA ATTATTTGAT
  TAGGATTTTG AAAAGATCAT TGGCTCTTCG TCATGCCGAT TGACACCCTC
  CACAAGCCAA GAGAAACTTA AGTTGTAAAC TTTCTCACTC CAAGCCTTCT
  ATATAAACAT GTATTGGATG TGAAGTTATT GCATAACTTG CATTGAACAA
  TAGAAAATAA CAAAAAAAAG TAAAAAAGTA GAAAGAAAT ATG,
```

24. A DNA fragment as claimed in claim 21, c h a r a c t e r i s e d  by being identical with, derived from or comprising a 5' flanking region of the Lbc$_2$ gene with the sequence:

```
  TCGAGTTTTT ACTGAACATA CATTTATTAA AAAAAACTCT CTAGTGTCCA
  TTTATTCGGC GAGAAGCCTT CTCGTGCTTT ACACACTTTA ATATTATTAT
  ATCCCCACCC CCACCAAAAA AAAAAAAACT GTTATATCTT TCCAGTACAT
  TTATTTCTTA TTTTTACAAA GGAAACTTCA CGAAAGTAAT TACAAAAAAG
  ATAGTGAACA TCATTTTTTT AGTTAAGATG AATTTTAAAA TCACACTTTT
  TTATATTTTT TTGTTACCCT TTTCATTATT GGGTGAAATC TCATAGTGAA
  ACTATTAAAT AGTTTGGGCT CAAGTTTTAT TAGTAAAGTC TGCATGAAAT
  TTAACTTAAT AATAGAGAGA GTTTTGGAAA GGTAACGAAT GTTAGAAAGT
  GTGATATTAT TATAGTTTTA TTTAGATTAA TAATTATGTT TACATGAAAA
  TTGACAATTT ATTTTTAAAA TTCAGAGTAA TACTTAAATT ACTTATTTAC
  TTTAAGATTT TGAAAAGATC ATTTGGCTCT TCATCATGCC GATTGACACC
  CTCCACAAGC CAAGAGAAAC TTAAGTTGTA ATTTTTCTAA CTCCAAGCCT
  TCTATATAAA CACGTATTGG ATGTGAAGTT GTTGCATAAC TTGCATTGAA
  CAATAGAAAT AACAACAAAG AAAATAAGTG AAAAAAGAAA TATG,
```

25. A DNA fragment as claimed in claim 21, c h a r a c t e r i s e d  by being identical with, derived from or comprising a 5' flanking region of the Lbc₃ gene with the sequence:

the Lbc$_3$ gene with the sequence:

```
TATGAAGATT AAAAAATACA CTCATATATA TGCCATAAGA ACCAACAAAA
GTACTATTTA AGAAAGAAA AAAAAAACCT GCTACATAAT TTCCAATCTT
GTAGATTTAT TTCTTTTATT TTTATAAAGG AGAGTTAAAA AAATTACAAA
ATAAAAATAG TGAACATCGT CTAAGCATTT TTATATAAGA TGAATTTTAA
AAATATAATT TTTTTGTCTA AATCGTATGT ATCTTGTCTT AGAGCCATTT
TTGTTTAAAT TGGATAAGAT CACACTATAA AGTTCTTCCT CCGAGTTTGA
TATAAAAAAA ATTGTTTCCC TTTTGATTAT TGGATAAAAT CTCGTAGTGA
CATTATATTA AAAAAATTAG GGCTCAATTT TTATTAGTAT AGTTTGCATA
AATTTTAACT TAAAAATAGA GAAAATCTGG AAAAGGGACT GTTAAAAAGT
GTGATATTAG AAATTTGTCG GATATATTAA TATTTTATTT TATATGGAAA
CTAAAAAAAT ATATATTAAA ATTTTAAATT CAGAATAATA CTTAAATTAT
TTATTTACTG AAAATGAGTT GATTTAAGTT TTTGAAAAGA TGATTGTCTC
TTCACCATAC CAATTGATCA CCCTCCTCCA ACAAGCCAAG AGAGACATAA
GTTTTATTAG TTATTCTGAT CACTCTTCAA GCCTTCTATA TAAATAAGTA
TTGGATGTGA AGTTGTTGCA TAACTTGCAT TGAACAATTA ATAGAAATAA
CAGAAAAGTA GAAAAGAAAT ATG.
```

26. A DNA fragment as claimed in claim 21, c h a r a c t e r i s e d by the DNA fragment comprising the inducible plant promoter being identical with, derived from or comprising 5' flanking regions of Lbc₃-5'-3'-CAT gene with the sequence:

regions of Lbc$_3$-5'-3'-CAT gene with the sequence:

```
TATGAAGATT AAAAAATACA CTCATATATA TGCCATAAGA ACCAACAAAA
GTACTATTTA AGAAAAGAAA AAAAAAACCT GCTACATAAT TTCCAATCTT
GTAGATTTAT TTCTTTTATT TTTATAAAGG AGAGTTAAAA AAATTACAAA
ATAAAAATAG TGAACATCGT CTAAGCATTT TTATATAAGA TGAATTTTAA
AAATATAATT TTTTTGTCTA AATCGTATGT ATCTTGTCTT AGAGCCATTT
TTGTTTAAAT TGGATAAGAT CACACTATAA AGTTCTTCCT CCGAGTTTGA
TATAAAAAAA ATTGTTTCCC TTTTGATTAT TGGATAAAAT CTCGTAGTGA
CATTATATTA AAAAAATTAG GGCTCAATTT TTATTAGTAT AGTTTGCATA
AATTTTAACT TAAAAATAGA GAAAATCTGG AAAAGGGACT GTTAAAAAGT
GTGATATTAG AAATTTGTCG GATATATTAA TATTTTATTT TATATGGAAA
CTAAAAAAAT ATATATTAAA ATTTTAAATT CAGAATAATA CTTAAATTAT
TTATTTACTG AAAATGAGTT GATTTAAGTT TTTGAAAAGA TGATTGTCTC
TTCACCATAC CAATTGATCA CCCTCCTCCA ACAAGCCAAG AGAGACATAA
GTTTTATTAG TTATTCTGAT CACTCTTCAA GCCTTCTATA TAAATAAGTA
TTGGATGTGA AGTTGTTGCA TAACTTGCAT TGAACAATTA ATAGAAATAA
CAGAAAAGTA GAATTCTAAA ATG
```

27. A DNA fragment as claimed in claim 19, c h a r a c t e r i s e d by being identical with,

76

derived from or comprising 5' flanking regions of
the N23 gene with the sequence:

```
         10        20        30        40        50        60        70
GAATTCGAGCTCGCCCGGGGATCGATCCTCTAGAGTCGACCTGCAGCCCAAGCTTGGATCAATCAATTAA
EcoRI                             SalI

         80        90       100       110       120       130       140
TTCTATTGAGACACGATTTGAACAATTTTTACATTATGAGACTATTTTTGGTTTTTTATTTGATCCAAAA

        150       160       170       180       190       200       210
AAATTTAAAGCTTTAGATGATGATGAATTGAANNAATATTGTATTAATNNTGAAAAGTTNNNNNGGTTTA

        220       230       240       250       260       270       280
ATGAATGCTATGATATTGATGGTCTTGATNTATTNNCAGAATTGAAAGTATTAAGAGAAGTGTTAAGAAA

        290       300       310       320       330       340       350
AGAAGTTAGCACACCAATAGAAGTATTGAGTTATATTAAAACTTTAGATTCTTTTCAAATGTTTACATTG

        360       370       380       390       400       410       420
CATATAGAATTTTATTGACAATCCTTATAACAGTTGCTACTGTTGAAAGACGTTCTTCAAAATTAAAATT

        430       440       450       460       470       480       490
ACTTAAATCATATCTAAAATCAACAATGTTACAAGATAGATTGAATGAGTTAGTTATTTTATCTATTGAA

        500       510       520       530       540       550       560
AGTAAAGTGTTAGAATTGTTTGATTATAAAACTCTGATAAATGATTTTGCAGTTAAAAAAACTAGAAGAT

        570       580       590       600       610       620       630
TAATATAAAAATTGATATTTTATATAATATATTAAGTCTCTTTAAAATTCTTGTAAAAAAAGACATTTTT

        640       650       660       670       680       690       700
AAATAATAAAATAAAGCAACTCTTAATTTTAATGAAACATCCCTTTGTTAAACCGAATCTTCCATAATGT

        710       720       730       740       750       760       770
AAAAATTAATGCTTGATGGAAGTTTTTAATTTGTTCTACTCAATACTCAAAGGGTTGTAAATATTTTTTT

        780       790       800       810       820       830       840
TATCATTTATATGTTGTAAATATGAATGCACTAGTAATTAGTTTAATGATAAAAATATATTCTACAGATAT

        850       860       870       880       890       900       910
ATTTCTGTCTCTTGGCAACTCGTGAGAATTGAATATATTATAAAGATGAAAGGTCGTTACAATTTTTTTT

        920       930       940       950       960       970       980
AGAATAAATATTTATATACAATTCCTAGATTTTGTTATAAAATTCACATATTGTATGAGTATAAATACAT

        990      1000      1010      1020      1030      1040      1050
GAGCACACACCAAACTAGTCTCAAATTAAGTAAGGTGCTAATTATTAGCGGCTAGCTAAGTAACCAAGTA
                                                        DdeI
```

ATTAATG

28.  A plasmid which can be used when carrying

77

out the method as claimed in claims 1-18, c h a r a c t e r i s e d by comprising a DNA fragment as claimed in any of the claims 19-27.

29. A plasmid as claimed in claim 28, c h a r- a c t e r i s e d by being pAR29.

30. A plasmid as claimed in claim 28, c h a r- a c t e r i s e d by being pAR30.

31. A plasmid as claimed in claim 28, c h a r- a c t e r i s e d by being pAR11.

32. A plasmid as claimed in claim 28, c h a r- a c t e r i s e d by being N23-CAT.

33. A transformant <u>Agrobacterium rhizogenes</u> 15834- strain which can be used when carrying out the method as claimed in any of the claims 1 to 18, c h a r a c t e r i s e d by the bacterium strain being transformed by a plasmid according to any of the preceding claims 28 to 32.

34. A transformant <u>Agrobacterium rhizogenes</u> 15834- strain which can be used when carrying out the method as claimed in any of the claims 1 to 18, c h a r a c t e r i s e d by the bacterium strain being transformed by pAR29 and being named AR1127.

35. A transformant <u>Agrobacterium rhizogenes</u> 15834- strain which can be used when carrying out the method as claimed in any of the claims 1 to 18, c h a r a c t e r i s e d by the bacterium strain being transformed by pAR30 and being named AR1134.

36. A transformant <u>Agrobacterium rhizogenes</u> 15834-strain which can be used when carrying out the method as claimed in any of the claims 1 to 18, c h a r a c t e r i s e d by the bacterium strain being transformed by pAR11 and being named AR1000.

37. A transformant <u>Agrobacterium rhizogenes</u> 15834-strain which can be used when carrying out the method as claimed in any of the claims 1 to 18, c h a r a c t e r i s e d by the bacterium strain being transformed by N23-CAT and being named AR204-N23-CAT.

38. Plants, parts of plants and plant cells, particularly of the family Leguminosae, obtainable by transformation with a recombinant DNA segment, fragment or plasmid according to any one of the claims 1 to 37.